# EUROPÄISCHE PATENTSCHRIFT

(11) **EP 3 270 767 B1**
(45) Veröffentlichungstag und Bekanntmachung des Hinweises auf die Patenterteilung: **18.11.2026**
(21) Anmeldenummer: 16711773.8
(22) Anmeldetag: 16.03.2016
(51) Int. Cl.: A61B 5/00, G01N 3/40, G01L 1/24, G01L 5/00, G02B 6/00, G02B 6/02

(54) **INDENTIERUNGSVORRICHTUNG MIT PIEZOELEKTRISCHEM STELLELEMENT**
INDENTATION DEVICE HAVING A PIEZOELECTRIC CONTROL ELEMENT
DISPOSITIF D'INDENTATION AVEC ÉLÉMENT DE COMMANDE PIÉZOÉLECTRIQUE

(30) Priorität: 17.03.2015 DE 102015003433; 17.03.2015 DE 102015003432
(43) Veröffentlichungstag der Anmeldung: 24.01.2018
(73) Patentinhaber: Hochschule München, 80335 München (DE)
(72) Erfinder: ROTHS, Johannes, 82223 Eichenau (DE); CLAUSEN-SCHAUMANN, Hauke, 82347 Bernried (DE); MARCHI, Gabriele, 81669 München (DE); ASZODI, Attila, 82152 Martinsried (DE)
(74) Vertreter: Lehmann, Horst Michael
(86) Internationale Anmeldenummer: PCT/EP2016/000475
(87) Internationale Veröffentlichungsnummer: WO 2016/146264

(56) Entgegenhaltungen:
- WO-A1-99/53292
- WO-A2-2012/015592
- MOERMAN KEVIN M ET AL: "A novel MRI compatible soft tissue indentor and fibre Bragg grating force sensor", MEDICAL ENGINEERING & PHYSICS, BUTTERWORTH-HEINEMANN, GB, vol. 35, no. 4, 20 July 2012 (2012-07-20), pages 486 - 499, XP028544537, ISSN: 1350-4533, DOI: 10.1016/J.MEDENGPHY.2012.06.014
- CHRISTOPHER R DENNISON ET AL: "An in-fiber Bragg grating sensor for contact force and stress measurements in articular joints;An in-fiber Bragg grating sensor for contact force and stress measurements in articular joints", MEASUREMENT SCIENCE AND TECHNOLOGY, IOP, BRISTOL, GB, vol. 21, no. 11, 6 October 2010 (2010-10-06), pages 115803, XP020199908, ISSN: 0957-0233, DOI: 10.1088/0957-0233/21/11/115803
- KIT MAN CHUNG ET AL: "Highly Sensitive Compact Force Sensor Based on Microfiber Bragg Grating", IEEE PHOTONICS TECHNOLOGY LETTERS, IEEE SERVICE CENTER, PISCATAWAY, NJ, US, vol. 24, no. 8, 1 April 2012 (2012-04-01), pages 700 - 702, XP011440353, ISSN: 1041-1135, DOI: 10.1109/LPT.2012.2187049

## Beschreibung

Die Erfindung betrifft eine Indentierungsvorrichtung zur Bestimmung von elastischen oder viskoelastischen Eigenschaften von Knorpelgewebe.

Der Artikel "A novel MRI compatible soft tissue indentor and fibre Bragg grating force sensor" von K. Möhrman et al., Medical Engineering & Physics, Butterworth-Heinemann, GB, Bd. 35, Nr. 4, 20. Juli 2012, Seiten 486 bis 499 beschreibt eine neuartige MRI-kompatible Indentierungsvorrichtung für weiche Gewebe sowie einen Faser-Bragg-Gitter-Kraftsensor. Der Kraftsensor für hohe Aufnahmerate (100 Hz) wurde für Kräfte bis zu 15 Newton kalibriert und zeigte einen maximalen Fehler von 0,043 Newton. Nachdem die Indentierungsvorrichtung und der Kraftsensor aus nicht-ferromagnetischen Materialien aufgebaut sind, sind sie MRI-kompatibel und es wurden keine Artefakte oder kurzfristige SNR-Verringerungen beobachtet. Das Indentierungssystem für weiche Gewebe umfasst einen hydraulischen Masterzylinder, der über eine Schlauchverbindung mit der Aktuatorvorrichtung gekoppelt ist. In dem MRI-Aktuator ist ein neuartiger MRI-kompatibler optischer Kraftsensor auf der Basis eines FBG eingebettet, der dynamische Kraftmessungen ermöglicht, die für viskoelastische Analyse und schnelle Detektion des Zeitverhaltens der Bewegung und der Krafteinwirkung geeignet sind.

Der Artikel "An in-fiber Bragg grating sensor for contact force and stress measurements in articular joints" von C. Dennison et al., Measurement Science and Technology, IOP, Bristol, GB, Bd. 21, Nr. 11, 6. Oktober 2010, Seite 115803 beschreibt einen Faser-Bragg-Gitter-Sensor für Messungen von Kontaktkraft und Druck in Gelenken. Der Kontaktkraftsensor und ein weiterer Bragg-Gitter-basierter Drucksensor (400 µm Durchmesser) werden dazu verwendet, gleichzeitige Messungen von Kontaktkraft und Flüssigkeitsdruck in intakten menschlichen Hüften von Verstorbenen durchzuführen. Es werden Verfahren vorgestellt, um die Bragg-Gitter-basierten Sensoren in das Gelenk einzusetzen, wobei die relevante Anatomie großteils intakt bleibt. Die Leistungsfähigkeit des Sensors wird unter Verwendung von numerischen Modellen vorhergesagt und die vorhergesagte Empfindlichkeit wird durch experimentelle Kalibrierungen überprüft.

Der Artikel "Highly Sensitive Compact Force Sensor Based on Microfiber Bragg Grating" von K. M. Chung et al., IEEE Photonics Technology Letters, IEEE Service Center, Piscataway, NJ, US, Bd. 24, Nr. 8, 1. April 2012, Seiten 700 bis 702 beschreibt einen hochempfindlichen kompakten Kraftsensor auf der Basis eines Mikrofaser-Bragg-Gitters. Es wird ein Miniatur-Kontaktkraftsensor basierend auf einem Mikrofaser-Bragg-Gitter von 30 µm Durchmesser präsentiert. Das Mikrofaser-Bragg-Gitter ist in ein konformes Elastomermaterial eingebettet, das eine extrem hohe Empfindlichkeit von 1,37 Millinewton (mN) und eine exzellente Linearität bis zu einer Kontaktkraft von 0,65 N aufweist. Dabei wird eine Mikrofaser von 30 µm Durchmesser im Zentrum eines Silikongummis eines Elastomermaterials eingebettet, welches dann innerhalb einer nicht-magnetisierten Edelstahlröhre angeordnet wird. Ein in die Mikrofaser eingeschriebenes FBG wird komprimiert, wenn es mit einem Objekt wie Blutgefäßen in Kontakt kommt, was zu einer Verschiebung der Reflexionswellenlänge des FBGs führt.

Die internationale Patentanmeldung WO 2012/015592 A2 beschreibt ein Verfahren und ein Gerät für eine Indentierung relativ zu einem Bezugspunkt ohne Verwendung einer Bezugssonde. Die Eindringtiefe wird relativ zu dem Instrument gemessen, das während einer Stoßbewegung, die in der Größenordnung von einer 1 Millisekunde abläuft, im Wesentlichen ortsfest bleibt. In einer Ausführungsform erzeugt eine Stoßbewegung mit einer Spitzenkraft der Größenordnung von 28 Newton eine Vertiefung in einem Knochen mit einer Tiefe von ungefähr 150 µm, während der sich das Instrumentengehäuse um weniger als eine 1 µs bewegt. Somit ist der Fehler bei der Messung der Eindringtiefe aufgrund der Bewegung des Gehäuses kleiner als 1%, was eine Bezugssonde unnötig macht. In einer Ausführungsform misst die Vorrichtung die Bruchbeständigkeit von harten Geweben, indem mikroskopische Frakturen in den harten Geweben erzeugt werden. Die Vorrichtung erzeugt diese mikroskopischen Frakturen, indem es mit einer scharfen Sonde auf die Probe einwirkt. Die Eindringtiefe in die Probe korreliert mit der Bruchbeständigkeit.

Die internationale Patentanmeldung WO 1999/053292 A1 beschreibt ein Instrument und ein Verfahren zum Messen der Kompressionseigenschaften eines Körpers (z.B. Gelenkknorpel). Das Instrument umfasst eine längliche Sonde, eine Vorspannanordnung zum Anlegen einer vorbestimmten Vorbelastung an den Körper, um eine Messreferenz bei der vorbestimmten Vorbelastung zu erzeugen, ein Stellglied zum Bewegen der länglichen Sonde, um eine Kompressionskraft an den Körper anzulegen und eine Messvorrichtung, die die Verschiebung der länglichen Sonde relativ zu der Messreferenz misst.

Es ist Aufgabe der Erfindung, eine Indentierungsvorrichtung zur Verfügung zu stellen, die eine genaue Bestimmung von elastischen oder viskoelastischen Eigenschaften eines Gewebes ermöglicht.

Die Aufgabe der Erfindung wird durch ein Indentierungsvorrichtung zur Bestimmung von elastischen oder viskoelastischen Eigenschaften von Knorpelgewebe gemäß Anspruch 1 gelöst.

Eine Indentierungsvorrichtung entsprechend den Ausführungsformen der Erfindung dient zur Bestimmung von elastischen oder viskoelastischen Eigenschaften von Knorpelgewebe. Die Indentierungsvorrichtung weist eine Messsonde zur Indentierung eines Gewebes auf, welche eine optische Faser umfasst. Des Weiteren umfasst die Indentierungsvorrichtung mindestens ein in die optische Faser eingeschriebenes Faser-Bragg-Gitter, das dazu vorgesehen ist, eine auf die Spitze der optischen Faser einwirkende Kraft zu detektieren. Die Indentierungsvorrichtung ist dazu ausgelegt, den Vorschub der Messsonde und die auf die Spitze der optischen Faser einwirkende Kraft zu erfassen. Die Messsonde umfasst ein Röhrchen, innerhalb dessen die optische Faser befestigt ist, wobei das Röhrchen einen Außendurchmesser von weniger als 1 Millimeter aufweist. Die Indentierungsvorrichtung weist ein piezoelektrisches Stellelement zur Positionierung der Messsonde auf, das mit der Messsonde mechanisch verbunden ist und das dazu ausgelegt ist, die Messsonde auf das zu vermessende Gewebe zu zu bewegen und in das Gewebe einzudrücken. Die Indentierungsvorrichtung ist dazu ausgelegt, die Spitze der optischen Faser in das zu untersuchende Gewebe einzudrücken.

Durch eine Indentierungsvorrichtung entsprechend den Ausführungsformen der vorliegenden Erfindung wird insbesondere für sehr kleine Indentierungstiefen von weniger als 10 µm eine genaue Bestimmung der elastischen Eigenschaften des untersuchten Gewebes ermöglicht. Dabei wird zur exakten Steuerung des Vorschubs der Messsonde ein piezoelektrisches Stellelement eingesetzt, das insbesondere bei kleinen Indentierungstiefen eine Steuerung des Vorschubs mit einer Genauigkeit im Sub-Mikrometerbereich ermöglicht. Das piezoelektrische Stellelement ermöglicht also insbesondere kleine Stellwege mit hoher Positioniergenauigkeit, kann aber darüber hinaus auch für größere Indentierungstiefen von beispielsweise einigen hundert Mikrometern oder mehr eingesetzt werden, so dass die Indentierungsvorrichtung wahlweise für Messungen mit sehr kleiner Indentierungstiefe oder für gröbere Indentierungsmessungen eingesetzt werden kann.

Diese hohe Positioniergenauigkeit bei der Steuerung des Vorschubs durch Verwendung eines piezoelektrischen Stellelements wird durch eine präzise Messung der Indentierungskraft ergänzt. Diese genaue Kraftmessung wird durch die Verwendung von mindestens einem in die Glasfaser eingeschriebenen Faser-Bragg-Gitter ermöglicht. Dabei wird die auf die Glasfaser einwirkende Indentierungskraft gemessen, indem die spektroskopische Verschiebung der Bragg-Wellenlänge des Faser-Bragg-Gitters verfolgt wird. Durch den Einsatz einer rein optischen Kraftmessmethode ist sichergestellt, dass die Indentierungsmessung nicht durch elektromagnetische Interferenz beeinträchtigt oder gestört werden kann. Darüber hinaus sind Glasfasern als Messsonden für minimalinvasive "in vivo"-Messungen am menschlichen Körper gut geeignet, unter anderem weil sie sich sehr gut sterilisieren lassen. Darüber hinaus wird durch den Einsatz von Glasfasern die Realisierung von sehr dünnen Messsonden erreicht, die perkutan (also durch die Haut hindurch) bis zum zu vermessenden Gewebe vorgeschoben werden können und eine minimalinvasive Bestimmung der elastischen oder viskoelastischen Eigenschaften des Gewebes, insbesondere beispielsweise eines Knorpelgewebes, ermöglichen.

Durch die Möglichkeit, Elastizitätsveränderungen in sehr dünnen Gewebeschichten zu erfassen und quantitativ zu bestimmen, eignet sich die vorgeschlagene Indentierungsvorrichtung insbesondere für eine Früherkennung der Arthrose. Durch die Vermessung von dünnen Oberflächenschichten von Knorpelgewebe und die Erkennung von degenerativen Veränderungen kann der Zeitpunkt, zu dem die Arthrose in der Früherkennungsdiagnose erkennbar wird, nach vorne verlagert werden. Dies ermöglicht eine frühzeitige Behandlung und eine dementsprechende Verzögerung des Krankheitsverlaufs.

Vorzugsweise ist die optische Faser in das Röhrchen der Messsonde eingeschoben und am Röhrchen befestigt. Vorzugsweise ist das Röhrchen der Messsonde dazu ausgelegt, die im Inneren des Röhrchens befestigte optische Faser zu stabilisieren und zu fixieren. Vorzugsweise handelt es sich bei dem Röhrchen der Messsonde um ein Metallröhrchen oder ein Glasröhrchen. Vorzugsweise weist das Röhrchen der Messsonde einen Außendurchmesser im Bereich von ca. 200 Mikrometer bis 500 Mikrometer auf. Vorzugsweise weist das Röhrchen der Messsonde einen Außendurchmesser im Bereich von ca. 200 Mikrometer bis 300 Mikrometer auf. Vorzugsweise weist das Röhrchen der Messsonde einen Innendurchmesser im Bereich von ca. 90 Mikrometer bis 170 Mikrometer auf. Vorzugsweise weist die optische Faser einen Außendurchmesser im Bereich von ca. 80 Mikrometer bis 125 Mikrometer auf.

Vorzugsweise weist die Indentierungsvorrichtung zusätzlich ein äußeres Führungsröhrchen für die Messsonde auf, wobei die Messsonde im äußeren Führungsröhrchen verschieblich aufgenommen ist.

Vorzugsweise weist die Indentierungsvorrichtung ein Gehäuse auf und das äußere Führungsröhrchen ist mit dem Gehäuse der Indentierungsvorrichtung mechanisch verbunden. Vorzugsweise ist die Messsonde angetrieben durch das piezoelektrische Stellelement und geführt im äußeren Führungsröhrchen zum Gewebe hin und vom Gewebe weg bewegbar. Vorzugsweise handelt es sich bei dem äußeren Führungsröhrchen um ein Metallröhrchen. Vorzugsweise ist das Röhrchen der Messsonde so dimensioniert, dass es im äußeren Führungsröhrchen verschieblich aufgenommen werden kann. Vorzugsweise weist das äußere Führungsröhrchen einen Außendurchmesser im Bereich von ca. 0,6 mm bis 1 mm auf.

Vorzugsweise ist das piezoelektrische Stellelement dazu ausgelegt, die Messsonde in Richtung auf das Gewebe zu und vom Gewebe weg zu positionieren. Vorzugsweise ist das piezoelektrische Stellelement dazu ausgelegt, die Messsonde auf das zu vermessende Gewebe zu und vom Gewebe weg zu bewegen. Vorzugsweise ist das piezoelektrische Stellelement dazu ausgelegt, die Messsonde in Richtung auf das Gewebe zu und vom Gewebe weg zu bewegen und dabei in das Gewebe einzudrücken. Vorzugsweise ist das piezoelektrische Stellelement dazu ausgelegt, in Abhängigkeit von einem Hochspannungssignal die Messsonde in Richtung auf das Gewebe zu und vom Gewebe weg zu positionieren.

Vorzugsweise ist zur Steuerung des Vorschubs an das piezoelektrische Stellelement eine Spannung von bis zu einigen hundert Volt anlegbar, wobei durch die angelegte Spannung ein Vorschub der Messsonde von bis zu einigen hundert Mikrometer bewirkt wird. Vorzugsweise handelt es sich bei dem piezoelektrischen Stellelement um ein rückgekoppeltes piezoelektrisches Stellelement, das dazu ausgelegt ist, die am piezoelektrischen Stellelement anliegende Spannung in Abhängigkeit vom tatsächlichen Vorschub des piezoelektrischen Stellelements einzuregeln. Vorzugsweise erfolgt die durch das piezoelektrische Stellelement bewirkte Positionierung der Messsonde mit einer Genauigkeit im Submikrometerbereich. Vorzugsweise ist der Vorschub der Messsonde während einer Indentierungsmessung so einstellbar, dass eine Indentierungstiefe von weniger als 40 Mikrometer erzielt wird. Vorzugsweise ist der Vorschub der Messsonde während einer Indentierungsmessung so einstellbar, dass eine Indentierungstiefe von weniger als 20 Mikrometer erzielt wird. Vorzugsweise ist der Vorschub der Messsonde während einer Indentierungsmessung so einstellbar, dass eine Indentierungstiefe von weniger als 10 Mikrometer erzielt wird.

Erfindungsgemäß weist die optische Faser ein erstes Faser-Bragg-Gitter auf, das dazu ausgelegt ist, die auf die Spitze der optischen Faser einwirkende Kraft zu detektieren.

Vorzugsweise ist das erste Faser-Bragg-Gitter im Bereich der Spitze der optischen Faser angeordnet. Vorzugsweise verursacht die auf die Spitze der optischen Faser einwirkende Kraft eine Stauchung des ersten Faser-Bragg-Gitters. Vorzugsweise wirkt während der Indentierung des Gewebes durch die Messsonde auf die Spitze der optischen Faser eine Kraft ein, die eine entsprechende Stauchung des ersten Faser-Bragg-Gitters hervorruft. Vorzugsweise verursacht die auf die Spitze der optischen Faser einwirkende Kraft eine Stauchung des ersten Faser-Bragg-Gitters, wobei die Stauchung des ersten Faser-Bragg-Gitters eine entsprechende Verschiebung der Mittenwellenlänge des vom ersten Faser-Bragg-Gitter reflektierten Lichts zu kleineren Wellenlängen hin bewirkt. Vorzugsweise verursacht die auf die Spitze der optischen Faser einwirkende Kraft eine Stauchung des ersten Faser-Bragg-Gitters, wobei die Stauchung des ersten Faser-Bragg-Gitters eine entsprechende Verschiebung der Bragg-Wellenlänge des ersten Faser-Bragg-Gitters zu kleineren Wellenlängen hin bewirkt. Vorzugsweise ist anhand einer Verschiebung der Wellenlänge des vom ersten Faser-Bragg-Gitter rückreflektierten Lichts die auf die Spitze der optischen Faser einwirkende Kraft bestimmbar.

Vorzugsweise ist die optische Faser in das Röhrchen der Messsonde eingeschoben und an mindestens einer Verklebungsstelle mit dem Röhrchen verklebt.

Vorzugsweise weist die optische Faser ein erstes Faser-Bragg-Gitter auf, das dazu ausgelegt ist, die auf die Spitze der optischen Faser einwirkende Kraft zu detektieren, wobei das erste Faser-Bragg-Gitter zwischen der vordersten der mindestens einen Verklebungsstelle und der Spitze der optischen Faser angeordnet ist.

Vorzugsweise weist die optische Faser im Bereich hinter der vordersten der mindestens einen Verklebungsstelle ein zweites Faser-Bragg-Gitter auf, das als Referenz-Faser-Bragg-Gitter zur Durchführung einer Temperaturkompensation für das erste Faser-Bragg-Gitter vorgesehen ist.

Vorzugsweise ist Licht von außen in die optische Faser einkoppelbar. Vorzugsweise ist von dem mindestens einen Faser-Bragg-Gitter rückreflektiertes Licht aus der optischen Faser auskoppelbar. Vorzugsweise erfolgt das Ein- und Auskoppeln von Licht in die optische Faser über Gradientenindexlinsen.

Vorzugsweise ist das piezoelektrische Stellelement dazu ausgelegt, die Messsonde während einer Indentierungsmessung mit einem linearen Vorschub in Richtung des Gewebes zu bewegen. Vorzugsweise ist die Indentierungsvorrichtung dazu ausgelegt, die während eines linearen Vorschubs der Messsonde auf die Spitze der optischen Faser einwirkende Kraft zu erfassen. Vorzugsweise umfasst die Indentierungsvorrichtung eine Spannungsversorgung, die dazu ausgelegt ist, die am piezoelektrischen Stellelement anliegende Spannung so zu erhöhen, dass die Messsonde mit einem linearen Vorschub auf das Gewebe zu bewegt und in das zu untersuchende Gewebe eingedrückt wird. Vorzugsweise umfasst die Indentierungsvorrichtung eine Spannungsversorgung, die dazu ausgelegt ist, dem piezoelektrischen Stellelement eine sägezahnförmige oder dreieckförmige Spannung zuzuführen.

Vorzugsweise umfasst die Indentierungsvorrichtung eine Auswerteeinheit, die dazu ausgelegt ist, die auf die optische Faser einwirkende Kraft in Abhängigkeit vom Vorschub der Messsonde zu erfassen.

Vorzugsweise ist die Auswerteeinheit dazu ausgelegt, aus dem Vorschub der Messsonde und der auf die Spitze der optischen Faser einwirkenden Kraft elastische oder viskoelastische Eigenschaften des Gewebes zu ermitteln. Vorzugsweise ist die Auswerteeinheit dazu ausgelegt, aus dem Zusammenhang zwischen dem Vorschub der Messsonde und der auf die Spitze der optischen Faser einwirkenden Kraft ein Elastizitätsmodul des untersuchten Gewebes zu bestimmen.

Vorzugsweise umfasst die Indentierungsvorrichtung eine Spannungsversorgung, die dazu ausgelegt ist, der am piezoelektrischen Stellelement anliegenden Spannung eine oszillierende Spannung zu überlagern, die eine oszillierende Bewegung der in das Gewebe eingedrückten Messsonde verursacht.

Vorzugsweise ist die Indentierungsvorrichtung dazu ausgelegt, die während der oszillierenden Bewegung der Messsonde auf die Spitze der optischen Faser einwirkende oszillierende Kraft zu erfassen. Vorzugsweise umfasst die Indentierungsvorrichtung eine Auswerteeinheit, die dazu ausgelegt ist, einen Phasenversatz zwischen der oszillierenden Bewegung der Messsonde und einer während der oszillierenden Bewegung der Messsonde auf die Spitze der optischen Faser einwirkenden oszillierenden Kraft zu bestimmen. Vorzugsweise umfasst die Indentierungsvorrichtung eine Auswerteeinheit, die dazu ausgelegt ist, einen Phasenversatz zwischen der oszillierenden Bewegung der Messsonde und einer während der oszillierenden Bewegung der Messsonde auf die Spitze der optischen Faser einwirkenden oszillierenden Kraft zu bestimmen und aus der Abhängigkeit des Phasenversatzes von der Frequenz der oszillierenden Bewegung ein Relaxationsverhaltens des Gewebes abzuleiten.

Vorzugsweise ist die Indentierungsvorrichtung als auseinandernehmbare Indentierungsvorrichtung realisiert. Vorzugsweise ist die Messsonde zu Reinigungszwecken vom Gehäuse lösbar. Vorzugsweise ist die Messsonde über eine lösbare mechanische Verbindung mit dem piezoelektrischen Stellelement verbunden. Vorzugsweise ist die Messsonde mittels eines Bajonett- oder Schraubverschlusses mit dem piezoelektrischen Stellelement verbunden. Vorzugsweise erfolgt das Ein- und Auskoppeln von Licht in die optische Faser über Gradientenindexlinsen.

Vorzugsweise weist die Indentierungsvorrichtung ein äußeres Führungsröhrchen für die Messsonde auf, wobei das äußere Führungsröhrchen zu Reinigungszwecken vom Gehäuse lösbar ist. Vorzugsweise weist die Indentierungsvorrichtung ein äußeres Führungsröhrchen für die Messsonde auf, wobei das äußere Führungsröhrchen über eine lösbare mechanische Verbindung mit dem Gehäuse der Indentierungsvorrichtung verbunden ist. Vorzugsweise weist die Indentierungsvorrichtung ein äußeres Führungsröhrchen für die Messsonde auf, wobei das äußere Führungsröhrchen mittels eines Bajonett- oder Schraubverschlusses mit dem Gehäuse der Indentierungsvorrichtung verbunden ist.

Erfindungsgemäß handelt es sich bei dem Gewebe um Knorpelgewebe. Erfindungsgemäß handelt es sich bei dem Gewebe um Knorpelgewebe, wobei die Indentierungsvorrichtung dazu ausgelegt ist, die elastischen oder viskoelastischen Eigenschaften des Knorpelgewebes zu bestimmen. Vorzugsweise ist die Indentierungsvorrichtung für eine minimalinvasive Diagnostik ausgelegt. Vorzugsweise ist die Indentierungsvorrichtung für eine Untersuchung von Gewebe in vivo ausgelegt. Vorzugsweise ist die Indentierungsvorrichtung für eine Untersuchung von Knorpelgewebe in vivo ausgelegt. Vorzugsweise handelt es sich bei dem Gewebe um Knorpelgewebe, wobei die Indentierungsvorrichtung dazu ausgelegt ist, anhand einer Veränderung von elastischen oder viskoelastischen Eigenschaften des Knorpelgewebes eine beginnende Degeneration des Knorpelgewebes festzustellen. Vorzugsweise ist die Indentierungsvorrichtung für eine Diagnose der Arthrose ausgelegt.

Eine Messanordnung entsprechend den Ausführungsformen der Erfindung dient zur Messung von elastischen oder viskoelastischen Eigenschaften eines Knorpelgewebes.

Die Messanordnung umfasst eine Indentierungsvorrichtung wie oben beschrieben sowie einen optischen Interrogator, der dazu ausgelegt ist, Anregungslicht in die optische Faser einzukoppeln und das von dem mindestens einen Faser-Bragg-Gitter rückreflektierte Licht aus der optischen Faser auszukoppeln und auszuwerten.

Vorzugsweise umfasst der optische Interrogator eine Lichtquelle, einen Faserkoppler und einen Fotodetektor.

Vorzugsweise handelt es sich bei der Lichtquelle um einen durchstimmbaren Laser oder um eine breitbandige Weißlichtquelle. Vorzugsweise ist der optische Interrogator dazu ausgelegt, Anregungslicht in die optische Faser einzukoppeln und das von dem mindestens einen Faser-Bragg-Gitter rückreflektierte Licht aus der optischen Faser auszukoppeln und auszuwerten.

Nachfolgend wird die Erfindung anhand mehrerer in der Zeichnung dargestellter Ausführungsbeispiele weiter beschrieben. Es zeigen
Fig. 1 eine schematische Darstellung einer Indentierungsmessung an einem Knorpel des Kniegelenks;
Fig. 2 einen Querschnitt durch ein Indentierungsmessgerät;
Fig. 3 eine Schrägbilddarstellung eines Indentierungsmessgeräts;
Fig. 4 eine vergrößerte Darstellung des stabförmigen Messelements des Indentierungsmessgeräts;
Fig. 5A eine Glasfaser, in die ein Faser-Bragg-Gitter eingeschrieben ist;
Fig. 5B die Variation des Brechungsindex n entlang der Längsausdehnung der Glasfaser;
Fig. 6A das Spektrum der einfallenden Lichtleistung P_{I} als Funktion der Wellenlänge λ;
Fig. 6B das Spektrum der reflektierten Lichtleistung P_{B} als Funktion der Wellenlänge λ;
Fig. 6C das Spektrum der transmittierten Lichtleistung P_{T} als Funktion der Wellenlänge λ;
Fig. 7 eine optische Messanordnung zur spektralen Analyse des rückreflektierten Lichts;
Fig. 8A eine Darstellung der Funktionsweise eines piezoelektrischen Stellelements;
Fig. 8B den Zusammenhang zwischen der an das piezoelektrische Stellelement angelegten Spannung U und der dadurch bewirkten Ausdehnung Δx des Piezos;
Fig. 9A die Position x der Messsondenspitze als Funktion der Zeit;
Fig. 9B die Verstimmung der Bragg-Wellenlänge λ_{B} als Funktion der Zeit;
Fig. 9C den Verlauf der Indentierungskraft F als Funktion der Zeit;
Fig. 10 eine schematische Darstellung der Geometrie einer Indentierungsmessung;
Fig. 11 ein Messverfahren, bei dem der Phasenversatz zwischen der sinusförmig variierten Position x der Messsondenspitze und der Indentierungskraft F erfasst und ausgewertet wird; und
Fig. 12 ein Messverfahren, bei dem sich ein linearer Vorschub der Messsondenspitze mit einer sinusförmigen Variation der Position abschnittsweise abwechselt.

Im Folgenden wird ein Indentierungsmessgerät beschrieben, mit dem die elastischen oder viskoelastischen Eigenschaften von Gewebe durch Eindrücken einer Messsonde in das Gewebe erfasst werden können. Mittels eines derartigen Indentierungsmessgeräts lassen sich insbesondere die elastischen oder viskoelastischen Eigenschaften von Knorpelgewebe bestimmen. Dies ist z.B. für die Diagnose der Arthrose von Bedeutung, bei der das Knorpelgewebe im Verlauf der Erkrankung immer weiter degeneriert. Darüber hinaus eignet sich das Indentierungsmessgerät jedoch auch zur Bestimmung der elastischen oder viskoelastischen Eigenschaften von anderen Geweben oder Substraten.

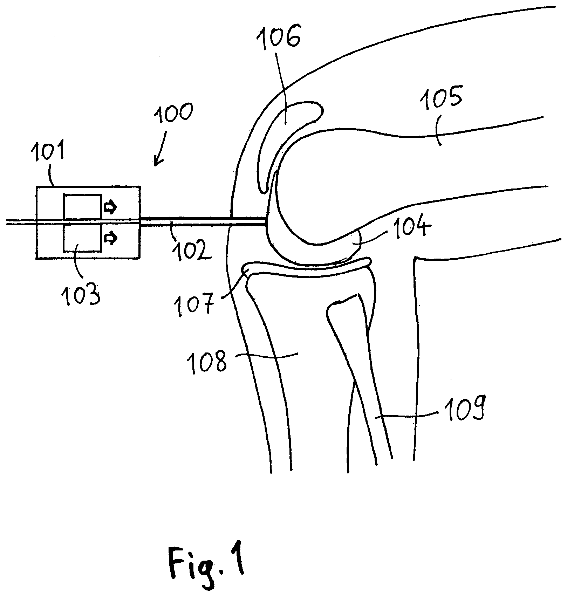

In Fig. 1 ist die prinzipielle Funktionsweise eines Indentierungsmessgeräts 100 schematisch gezeigt. Das Indentierungsmessgerät 100 umfasst ein Gehäuse 101 mit einem stabförmigen Messelement 102. In dem Gehäuse 101 ist ein piezoelektrisches Stellelement 103 untergebracht. Das stabförmige Messelement 102 umfasst eine Messsonde, die durch das piezoelektrische Stellelement 103 auf das Gewebe zu und vom Gewebe weg bewegt werden kann. Während des Vorschubs wird die auf die Messsonde einwirkende Kraft erfasst, und aus dem Zusammenhang zwischen Vorschub und Kraft können dann die elastischen oder viskoelastischen Eigenschaften des Gewebes hergeleitet werden.

In Fig. 1 wird das Indentierungsmessgerät 100 zur Bestimmung der elastischen oder viskoelastischen Eigenschaften eines Gelenkknorpels 104 am Oberschenkelknochen 105 des menschlichen Kniegelenks eingesetzt. In Fig. 1 sind darüber hinaus die Kniescheibe 106, der Meniskus 107, das Schienbein 108 und das Wadenbein 109 zu erkennen.

Mittels des Indentierungsmessgeräts 100 können die elastischen oder viskoelastischen Eigenschaften des Gelenkknorpels 104 minimalinvasiv bestimmt werden. Hierzu wird das stabförmige Messelement 102 mit der Messsonde durch die Haut hindurch bis zur Oberfläche des Gelenkknorpels 104 vorgeschoben. Anschließend wird die Indentierungsmessung in vivo vorgenommen. Die Messsonde wird durch das piezoelektrische Stellelement 103 vorgeschoben und in den Gelenkknorpel 104 eingedrückt. Während des Eindrückens wird die Kraft erfasst, die beim Eindrücken auf die Messsonde einwirkt. Aus dem Zusammenhang zwischen Vorschub und Kraft können dann die elastischen oder viskoelastischen Eigenschaften des Gelenkknorpels 104 hergeleitet werden.

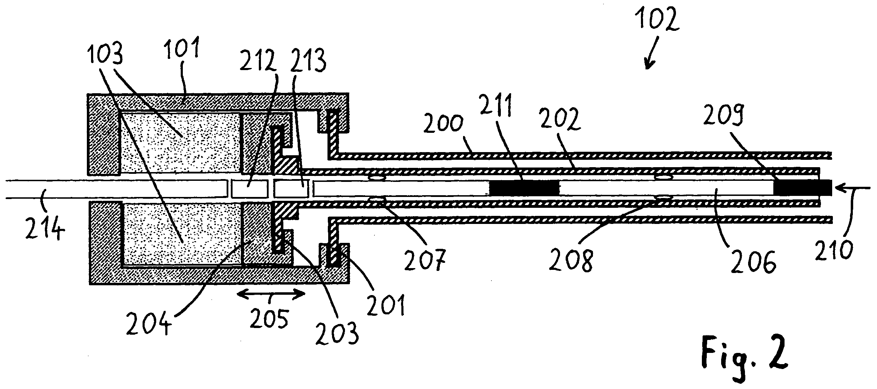

In Fig. 2 ist einen Querschnitt durch ein Indentierungsmessgerät gezeigt, wobei insbesondere die Struktur des stabförmigen Messelements 102 erkennbar ist. Das stabförmige Messelement 102 umfasst ein äußeres Führungsröhrchen 200, welches vorzugsweise aus Metall besteht. Das äußere Führungsröhrchen 200 wird am Gehäuse 101 befestigt. Hierzu kann das äußere Führungsröhrchen 200 z.B. mittels eines äußeren Flansches 201 in einem Bajonett- oder Schraubverschluss am vorderen Ende des Gehäuses 101 befestigt werden. Das äußere Führungsröhrchen 200 weist vorzugsweise einen Außendurchmesser von z.B. 0,6 mm bis 1 mm auf, wobei eventuell auch kleinere Außendurchmesser möglich wären. Je kleiner der Außendurchmesser des äußeren Führungsröhrchens 200 ist, desto besser eignet sich das Indentierungsmessgerät für die minimalinvasive Diagnostik.

Das äußere Führungsröhrchen 200 dient als Führungshülse für die eigentliche Messsonde, die innerhalb des äußeren Führungsröhrchens 200 verschieblich gelagert ist. Die Messsonde besteht aus einem Röhrchen 202 und einer im Inneren des Röhrchens 202 angebrachten ersten Glasfaser 206, die zur Erfassung der beim Eindrücken der Messsonde in das Gewebe auftretenden Kraft dient.

Das Röhrchen 202 besteht vorzugsweise aus Metall und kann z.B. einen Außendurchmesser von 250 µm und einen Innendurchmesser von 140 µm aufweisen. Das Röhrchen 202 weist einen inneren Flansch 203 auf. Über den inneren Flansch 203 kann das Röhrchen 202 z.B. mittels eines Bajonett- oder Schraubverschlusses mit einer Halterungsscheibe 204 mechanisch verbunden werden, die ihrerseits mit der Stirnseite des piezoelektrischen Stellelements 103 verbunden ist. Angetrieben durch das piezoelektrische Stellelement 103 wird die Halterungsscheibe 204 mit der daran befestigten Messsonde hin- und herbewegt, wie dies durch den Doppelpfeil 205 veranschaulicht ist. Dadurch wird die Messsonde innerhalb des äußeren Führungsröhrchens 200 hin- und herbewegt. Auf diese Weise ist es möglich, die Messsonde mittels des piezoelektrischen Stellelements 103 aus dem äußeren Führungsröhrchen 200 herauszubewegen und in das zu vermessende Gewebe einzudrücken.

Die Messsonde umfasst das Röhrchen 202 und die darin angebrachte erste Glasfaser 206. Die erste Glasfaser 206 ist als Kraftaufnehmer ausgebildet und dient zur Erfassung der beim Eindrücken der Messsonde in das Gewebe auftretenden Kraft. Die erste Glasfaser 206 kann beispielsweise einen Außendurchmesser von ca. 125 µm aufweisen. Da das Röhrchen 202 z.B. einen Innendurchmesser von 140 µm aufweist, kann die erste Glasfaser in das Röhrchen 202 eingeschoben werden. Die erste Glasfaser 206 wird mit Hilfe einer hinteren Verklebung 207 und einer vorderen Verklebung 208 mechanisch mit dem Röhrchen 202 verbunden. Auf diese Weise wird die erste Glasfaser 206 durch das Röhrchen 202 fixiert und stabilisiert.

Zur Bestimmung der elastischen Eigenschaften des jeweiligen Gewebes wird die beim Eindrücken auf die Spitze der ersten Glasfaser 206 wirkende Kraft 210 erfasst. Die Bestimmung dieser Kraft 210 erfolgt bei dem in Fig. 2 gezeigten Indentierungsmessgerät mit Hilfe von einem oder mehreren Faser-Bragg-Gittern, die in die erste Glasfaser 206 eingeschrieben sind. Derartige Faser-Bragg-Gitter verursachen eine Reflexion des einfallenden Lichts bei einer Bragg-Wellenlänge λ_{B}. Wenn die Kraft 210 auf die erste Glasfaser 206 einwirkt, bewirkt diese Kraft 210 eine Stauchung der ersten Glasfaser 206, was zu einer entsprechenden Verringerung der Gitterkonstante des Faser-Bragg-Gitters führt. Durch diese Stauchung verschiebt sich die Wellenlänge λ_{B} des reflektierten Lichts. Diese Verschiebung der Bragg-Wellenlänge stellt ein Maß für die auf die erste Glasfaser 206 einwirkende Kraft 210 dar.

Bei dem in Fig. 2 gezeigten Beispiel ist in der Nähe der Spitze der ersten Glasfaser 206 ein erstes Faser-Bragg-Gitter 209 eingeschrieben. Wenn nun beim Indentieren eines Gewebes die Kraft 210 auf die Spitze der ersten Glasfaser 206 einwirkt, dann wird das erste Faser-Bragg-Gitter 209 infolge dieser Krafteinwirkung gestaucht. Diese Stauchung des ersten Faser-Bragg-Gitters 209 kann anhand der Verschiebung der reflektierten Wellenlänge detektiert und ausgewertet werden. Dadurch ist es möglich, die während der Indentierung auf die Spitze der ersten Glasfaser 206 einwirkende Kraft 210 in ihrem zeitlichen Verlauf zu erfassen.

Zusätzlich zum ersten Faser-Bragg-Gitter 209 kann zwischen der hinteren Verklebung 207 und der vorderen Verklebung 208 ein zweites Faser-Bragg-Gitter 211 angeordnet sein, dessen Gitterkonstante sich von der Gitterkonstante des ersten Faser-Bragg-Gitters 209 unterscheidet. Die Wellenlänge des vom zweiten Faser-Bragg-Gitter 211 reflektierten Lichts unterscheidet sich daher von der Wellenlänge, die vom ersten Faser-Bragg-Gitter 209 reflektiert wird. Da das zweite Faser-Bragg-Gitter 211 hinter der vorderen Verklebung 208 angeordnet ist, wird es durch die auf die Sondenspitze einwirkende Kraft nicht gestaucht. Das zweite Faser-Bragg-Gitter 211 ist so angeordnet, dass es dazu verwendet werden kann, die Temperaturabhängigkeit der von den beiden Faser-Bragg-Gittern 209, 211 reflektierten Wellenlängen zu erfassen und rechnerisch die Temperaturabhängigkeit des ersten Faser-Bragg-Gitters 209 zu kompensieren. Das zweite Faser-Bragg-Gitter 211 dient also zur Durchführung einer Referenzmessung bei einer zweiten Wellenlänge, die nur von der Temperatur, nicht aber von der einwirkenden Kraft 210 abhängt.

Dabei ist es von Vorteil, wenn das zweite Faser-Bragg-Gitter 211 dicht hinter der vorderen Verklebung 208 angeordnet ist, also möglichst weit vorn im vorderen Bereich der ersten Glasfaser 206. Dadurch ist gewährleistet, dass nach dem Einschieben des stabförmigen Messelements 102 in den Körper des Patienten auch das zweite Faser-Bragg-Gitter 211 möglichst rasch auf Körpertemperatur erwärmt wird und somit auf dieselbe Temperatur wie das erste Faser-Bragg-Gitter 209 gebracht wird.

Bei der in Fig. 2 gezeigten Lösung ist die erste Glasfaser 206 über zwei Gradientenindex-Linsen bzw. GRIN-Linsen 212, 213 mit einer zweiten Glasfaser 214 optisch gekoppelt. Über die zweite Glasfaser 214 und die GRIN-Linsen 212, 213 kann Licht in die erste Glasfaser 206 eingekoppelt werden. Gewisse spektrale Teilbereiche dieses Lichts werden durch das erste Faser-Bragg-Gitter 209 und das zweite Faser-Bragg-Gitter 211 zurückreflektiert. Die zurückreflektierten Lichtkomponenten können über die beiden GRIN-Linsen 212, 213 und die zweite Glasfaser 214 ausgekoppelt und spektral analysiert werden.

Die optische Kopplung zwischen der ersten Glasfaser 206 und der zweiten Glasfaser 214 über die beiden GRIN-Linsen 212, 213 ermöglicht es, das in Fig. 2 gezeigte Indentierungsmessgerät auseinanderzubauen. Zum Auseinanderbauen des Indentierungsmessgeräts wird zunächst das äußere Führungsröhrchen 200 abgenommen. Hierzu wird der äußere Flansch 201 aus dem Bajonett- oder Schraubverschluss am Gehäuse 101 gelöst, anschließend kann das äußere Führungsröhrchen 200 abgezogen werden. Als nächstes wird die Messsonde abgenommen. Hierzu wird der innere Flansch 203 aus dem Bajonett- oder Schraubverschluss an der Halterungsscheibe 204 gelöst. Anschließend kann das Röhrchen 202 zusammen mit der darin befindlichen GRIN-Linse 213 und der eingeklebten ersten Glasfaser 206 abgenommen werden. Dadurch ist es möglich, sämtliche Bestandteile des stabförmigen Messelements 102 zu reinigen und zu desinfizieren.

Nach der Reinigung wird das Indentierungsmessgerät wieder zusammengebaut. Hierzu wird zunächst der innere Flansch 203 des Röhrchens 202 in den zugehörigen Bajonett- oder Schraubverschluss der Halterungsscheibe 204 eingedreht. Anschließend wird das äußere Führungsröhrchen 200 auf das Röhrchen 202 aufgeschoben, und der äußere Flansch 201 wird in den zugehörigen Bajonett- oder Schraubverschluss am vorderen Ende des Gehäuses 101 eingedreht.

Bei dem in Fig. 2 gezeigten Indentierungsmessgerät wird zur Erfassung der Indentierungskraft eine optische Kraftdetektion mit Hilfe von einem oder mehreren Faser-Bragg-Gittern eingesetzt. Dabei wird die optische Faser sowohl als Sensorelement als auch zur Signalübertragung genutzt. Die Signalgewinnung und -übertragung erfolgt daher ohne den Einsatz von Elektrizität und kann daher durch elektromagnetische Störungen nicht beeinträchtigt werden. Dies ist insbesondere von Bedeutung bei der Anwesenheit von starken magnetischen Feldern, wie sie beispielsweise innerhalb von Magnetresonanztomographen zum Einsatz kommen.

Ein weiterer Vorteil ist, dass die Glasfasern einen sehr geringen Durchmesser von beispielsweise 125 µm aufweisen, so dass eine minimalinvasive Diagnostik ermöglicht wird. Es sind auch Spezialfasern erhältlich, die noch kleinere Faserdurchmesser aufweisen (beispielsweise 80 µm). Das Glasmaterial der Sensorfasern ist biokompatibel. Wie weiter oben beschrieben, kann das Indentierungsmessgerät auf einfache Weise in seine Bestandteile zerlegt werden, so dass die Komponenten des stabförmigen Messelements 102 gereinigt werden können. Ein weiterer Vorteil bei der Verwendung von Faser-Bragg-Gittern ist, dass in eine einzige optische Faser unterschiedliche Faser-Bragg-Gitter eingeschrieben werden können, welche Licht von unterschiedlicher Wellenlänge reflektieren. Durch einen derartigen Wellenlängenmultiplex können mehrere Faser-Bragg-Gitter in die gleiche Glasfaser integriert werden, so dass beispielsweise Temperatur und Dehnung getrennt voneinander erfasst werden können.

Bei dem in Fig. 2 gezeigten Indentierungsmessgerät wird ein piezoelektrisches Stellelement 103 als Vortrieb für die Messsonde verwendet. Ein derartiges piezoelektrisches Stellelement 103 ermöglicht einen Vorschub der als Kraftaufnehmer dienenden ersten Glasfaser 206 im Bereich von ca. 0 bis 500 µm mit einer Stellgenauigkeit im Sub-Mikrometerbereich. Diese hohe Positionierungsgenauigkeit ermöglicht es, bereits kleine Veränderungen der elastischen oder viskoelastischen Eigenschaften an der Oberfläche eines Gewebes zu detektieren. Dies ist insbesondere bei der Diagnose der Arthrose von Bedeutung, wo im Frühstadium zunächst eine leichte Veränderung der elastischen oder viskoelastischen Eigenschaften in einer dünnen Oberflächenschicht des Knorpelgewebes auftritt.

Der Verlauf der Arthrose wird in vier unterschiedliche Stadien eingeteilt. Das erste Stadium der Arthrose ist durch Rauigkeiten und eine Ausdünnung der Knorpelschicht gekennzeichnet. Durch die hohe Stellgenauigkeit, die durch das piezoelektrische Stellelement 103 ermöglicht wird, können sehr hoch aufgelöste Indentierungen mit weniger als 10 µm Eindringtiefe durchgeführt werden. Im Unterschied zu herkömmlichen Indentoren des Stands der Technik, die typischerweise Indentierungstiefen von 80 µm und mehr aufweisen, wird durch den piezoelektrischen Antrieb eine Untersuchung von Oberflächenbereichen in einem Dickenbereich von weniger als 10 µm ermöglicht. Dabei können die in diesen dünnen Oberflächenschichten auftretenden Veränderungen der Elastizitätseigenschaften durch die simultane Erfassung von Position und Kraft aufgelöst werden.

Dies ermöglicht es, eine Degradation der äußersten Schichten des Knorpels zu einem früheren Zeitpunkt zu erkennen, als dies mit den bisherigen deutlich gröberen Messverfahren möglich war. Je früher die Krankheit erkannt werden kann, desto besser kann die Degeneration des Knorpels verzögert werden, so dass die Lebensqualität des Patienten länger erhalten werden kann. Da es sich bei dem hier vorgestellten Indentierungsverfahren um eine minimalinvasive Prozedur handelt, ist der diagnostische Eingriff für den Patienten mit vergleichsweise geringen Belastungen verbunden.

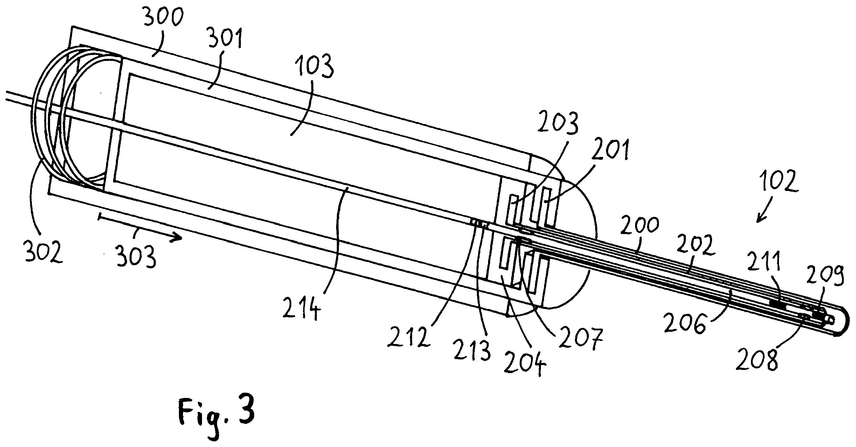

In Fig. 3 ist ein Beispiel eines Indentierungsmessgeräts in Schrägbildansicht gezeigt, wobei gleiche oder funktionell entsprechende Merkmale mit denselben Bezugszeichen wie in Fig. 1 und Fig. 2 versehen sind.

Bei dem in Fig. 3 gezeigten Indentierungsmessgerät ist das Gehäuse als zweiteiliges Gehäuse ausgebildet und umfasst einen stationären Teil 300 sowie einen beweglichen Teil 301, der innerhalb des stationären Teils 300 beweglich gelagert ist. Am hinteren Ende des stationären Teils 300 ist eine Feder 302 angeordnet, die den beweglichen Teil 301 des Gehäuses nach vorne drückt. Während der Durchführung der Indentierungsmessung hält die Bedienperson das Indentierungsmessgerät am stationären Teil 300 fest und drückt das stabförmige Messelement 102 gegen das zu vermessende Gewebe. Dabei wird der bewegliche Teil 301 mit dem stabförmigen Messelement 102 durch die Feder 302 mit einer konstanten Anpresskraft 303 gegen das zu vermessende Gewebe gedrückt. Dadurch liegt der vordere Bereich des äußeren Führungsröhrchens 200 mit konstanter Anpresskraft 303 am zu vermessenden Gewebe an. Dadurch werden definierte und reproduzierbare Ausgangsbedingungen für die Durchführung der Indentierungsmessung geschaffen.

Das äußere Führungsröhrchen 200 ist über den äußeren Flansch 201 am vorderen Ende des beweglichen Teils 301 des Gehäuses befestigt. Im Inneren des äußeren Führungsröhrchens 200 ist die Messsonde angeordnet, die das Röhrchen 202 und die darin befestigte erste Glasfaser 206 umfasst. Die Messsonde kann mittels des piezoelektrischen Stellelements 103 innerhalb des äußeren Führungsröhrchens 200 vor- und zurückbewegt werden. Dazu ist der innere Flansch 203 des Röhrchens 202 an der Halterungsscheibe 204 befestigt, die ihrerseits mit der Stirnseite des piezoelektrischen Stellelements 103 verbunden ist. Innerhalb des Röhrchens 202 ist die erste Glasfaser 206 angebracht, die über die beiden Verklebungen 207, 208 mit dem Röhrchen 202 verbunden ist. Dadurch wird die erste Glasfaser 206 durch das Röhrchen 202 fixiert und stabilisiert. Das erste Faser-Bragg-Gitter 209 ist zwischen der vorderen Verklebung 208 und der Spitze der ersten Glasfaser 206 angeordnet. Wenn die Spitze der ersten Glasfaser 206 gegen das zu vermessende Gewebe gedrückt wird, wird das erste Faser-Bragg-Gitter 209 entsprechend gestaucht, und die Wellenlänge des rückreflektierten Lichts verschiebt sich. Das erste Faser-Bragg-Gitter 209 dient daher zur Bestimmung der Indentierungskraft.

Das zweite Faser-Bragg-Gitter 211 ist hinter der vorderen Verklebung 208 angeordnet und wird daher durch die bei der Indentierung einwirkende Kraft nicht gestaucht. Das zweite Faser-Bragg-Gitter 211 dient als Referenzsensor und kann zur Kompensation der Temperaturabhängigkeit eingesetzt werden.

Über die beiden GRIN-Linsen 212, 213 ist die erste Glasfaser 206 mit einer zweiten Glasfaser 214 optisch gekoppelt. Über die zweite Glasfaser 214 kann Licht in die erste Glasfaser 206 eingekoppelt werden. Darüber hinaus kann das von den Faser-Bragg-Gittern 209, 211 rückreflektierte Licht über die zweite Glasfaser 214 ausgekoppelt werden.

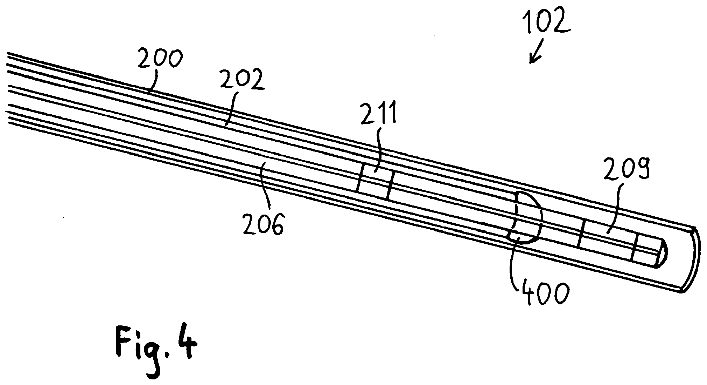

In Fig. 4 ist das vordere Ende des stabförmigen Messelements 102 noch einmal vergrößert herausgezeichnet. Zu erkennen sind das äußere Führungsröhrchen 200, das Röhrchen 202 sowie die erste Glasfaser 206. Im Unterschied zu Fig. 3 befindet sich die vordere Verklebung 400 in Fig. 4 unmittelbar am vorderen Ende des Röhrchens 202. Das erste Faser-Bragg-Gitter 209 befindet sich zwischen der vorderen Verklebung 400 und dem vorderen Ende der ersten Glasfaser 206, während das zweite Faser-Bragg-Gitter 211 hinter der vorderen Verklebung 400 angeordnet ist.

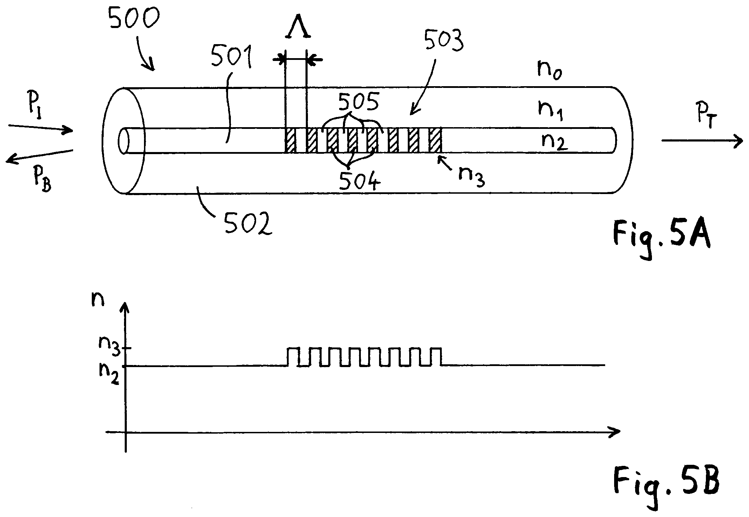

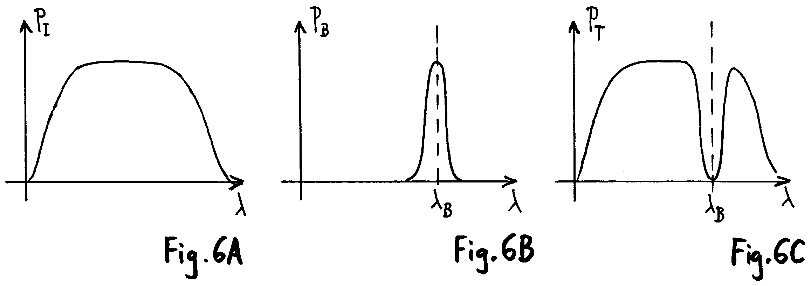

Im Folgenden soll anhand der Fig. 5A und Fig. 5B sowie Fig. 6A bis 6C die Struktur und Funktionsweise eines Faser-Bragg-Gitters beschrieben werden. Hierzu ist in Fig. 5A eine Glasfaser 500 gezeigt, die einen Faserkern (core) 501 sowie einen Mantel (cladding) 502 aufweist, wobei der Mantel 502 den Faserkern 501 rundum umschließt. Der Brechungsindex n₂ des Faserkerns 501 ist dabei größer als der Brechungsindex n₁ des Mantels 502, so dass also gilt: n₂ > n₁. Durch diese Bedingung wird eine Lichtausbreitung innerhalb der Glasfaser 500 ermöglicht.

Die in Fig. 5A gezeigte Glasfaser 500 weist ein Faser-Bragg-Gitter 503 auf. Als Faser-Bragg-Gitter bezeichnet man ein in die Faser eingeschriebenes optisches Interferenzfilter, das eine periodische Abfolge von Bereichen 504 mit hohem Brechungsindex und Bereichen 505 mit niedrigem Brechungsindex aufweist. Die Bereiche 504 mit hohem Brechungsindex n₃ sind in Fig. 5A schraffiert eingezeichnet. Die dazwischenliegenden Bereiche 505 weisen einen niedrigeren Brechungsindex n₂ auf. Als Gitterperiode Λ bezeichnet man den Abstand zwischen aufeinanderfolgenden Bereichen mit hohem Brechungsindex n₃.

In Fig. 5B ist die Variation des Brechungsindex n entlang der Längsrichtung der Glasfaser 500 aufgetragen. Anhand von Fig. 5B ist erkennbar, dass die schraffiert eingezeichneten Bereiche 504 einen Brechungsindex n₃ aufweisen, der höher ist als der Brechungsindex n₂ der dazwischenliegenden Bereiche 505.

Das Faser-Bragg-Gitter 503 wirkt als optisches Interferenzfilter, das einfallendes Licht einer bestimmten Wellenlänge reflektiert. Die Mittenwellenlänge des rückreflektierten Lichts wird als "Bragg-Wellenlänge" λ_{B} bezeichnet. Die Bragg-Wellenlänge λ_{B} des Faser-Bragg-Gitters ergibt sich aus der Bragg-Bedingung: ${λ}_{B} = {n}_{eff} ⋅ 2 Λ$ (1)

In dieser Formel bezeichnet λ_{B} die Wellenlänge des rückreflektierten Lichts im Vakuum, Λ die Gitterperiode des Faser-Bragg-Gitters und n_{eff} den effektiven Brechungsindex. Der effektive Brechungsindex n_{eff} hängt von der Geometrie (Kern- und Manteldurchmesser) des Wellenleiters, von den Brechungsindizes n₁, n₂, n₃ und von den Wellenmoden ab. Die spektrale Breite des rückreflektierten Lichts hängt von der Länge des Faser-Bragg-Gitters und der Stärke der Brechungsindexänderung zwischen den benachbarten Brechungsindexbereichen ab.

Zur Veranschaulichung der Bragg-Beziehung kann man sich vorstellen, dass das Faser-Bragg-Gitter aus vielen aufeinanderfolgenden Abschnitten der Länge Λ = λ/2 zusammengesetzt ist. Jeder Abschnitt der Länge Λ = λ/2 setzt sich aus einem ersten λ/4-Abschnitt mit hohem Brechungsindex n₃ und einem zweiten λ/4-Abschnitt mit niedrigem Brechungsindex n₂ zusammen. An jeder Grenzfläche zwischen den Abschnitten wird ein Teil der eingespeisten Amplitude durch die Fresnel-Reflexion reflektiert, so dass die reflektierte Welle am Ende jedes λ/4-Abschnitts entweder einen Phasensprung von 0° oder von 180° erfährt. Wenn die Bragg-Bedingung erfüllt ist, kommt es infolge der Mehrfachreflexion an den verschiedenen Grenzflächen bei der reflektierten Welle zu konstruktiver Interferenz, und die an den einzelnen Grenzflächen rückreflektierten Teilamplituden überlagern sich zu einer reflektierten Welle.

In den Fig. 6A, 6B und 6C sind die einfallende Leistung P_{I}, die reflektierte Leistung P_{B} und die durchgelassene Leistung P_{T} jeweils als Funktion der Wellenlänge aufgetragen.

Fig. 6A zeigt das Spektrum der einfallenden Leistung P_{I} als Funktion der Wellenlänge λ. Es ist zu erkennen, dass das einfallende Licht relativ breitbandig ist und eine Vielzahl von unterschiedlichen Lichtwellenlängen umfasst. Als Lichtquelle kann beispielsweise eine Weißlichtquelle eingesetzt werden.

Fig. 6B zeigt das Spektrum der vom Faser-Bragg-Gitter 503 reflektierten Leistung P_{B} als Funktion der Wellenlänge λ. Es ist zu erkennen, dass Wellenlängen, die innerhalb der Filterbandbreite um die Bragg-Wellenlänge λ_{B} liegen, vom Faser-Bragg-Gitter 503 zurückreflektiert werden.

Fig. 6C zeigt das Spektrum der durchgelassenen Leistung P_{T} als Funktion der Wellenlänge λ. Es ist zu erkennen, dass sowohl der Teil des einfallenden Spektrums unterhalb der Bragg-Wellenlänge λ_{B} als auch der Teil des einfallenden Spektrums oberhalb der Bragg-Wellenlänge λ_{B} durchgelassen werden, wohingegen diejenigen Wellenlängen, die innerhalb der Filterbandbreite um λ_{B} liegen, nicht durchgelassen werden. Diese Wellenlängen fehlen daher in der durchgelassenen Leistung P_{T}.

Das Faser-Bragg-Gitter 503 kann beispielsweise mit Hilfe von UV-Licht in die Glasfaser 500 eingeschrieben werden, um auf diese Weise die charakteristische Abfolge von Bereichen mit hohem und niedrigem Brechungsindex zu erzeugen. Für die Herstellung des Faser-Bragg-Gitters 503 kann beispielsweise ein Excimerlaser mit einer Wellenlänge λ = 248 nm verwendet werden. Durch das UV-Licht des Excimerlasers kann die für das Faser-Bragg-Gitter charakteristische Modulation des Brechungsindex innerhalb des Faserkerns 501 der Glasfaser 500 erzeugt werden.

Die Mittenwellenlänge λ_{B} der Filterbandbreite eines Faser-Bragg-Gitters wird sowohl durch eine mechanische Stauchung oder Dehnung als auch durch eine Temperaturänderung beeinflusst. Wenn beispielsweise infolge einer Krafteinwirkung eine Stauchung des Faser-Bragg-Gitters auftritt, dann verringert sich die Gitterperiode Λ des Faser-Bragg-Gitters, und dementsprechend verringert sich auch die Bragg-Wellenlänge λ_{B}.

Eine Temperaturänderung dagegen führt zu zwei unterschiedlichen Effekten: zum einen führt die thermische Ausdehnung zu einer Veränderung der Gitterperiode, die durch den thermischen Ausdehnungskoeffizienten α der Glasfaser beschrieben werden kann. Zum andern besitzt der Brechungsindex n eine Temperaturabhängigkeit, so dass eine Temperaturänderung auch zu einer Änderung der Brechungsindizes n₁, n₂ und n₃ und somit auch von n_{eff} führt. Beide Effekte tragen zur Temperaturabhängigkeit der Bragg-Wellenlänge λ_{B} bei. Insgesamt kann die Verschiebung Δλ_{B} der Bragg-Wellenlänge in Abhängigkeit von der Dehnung ε und der Temperaturänderung ΔT durch folgende Formel beschrieben werden: $\frac{{Δλ}_{B}}{{λ}_{B}} = \left(1-{p}_{e}\right) ⋅ ε + \left(α+ξ\right) ⋅ ΔT$ (2)

In dieser Formel bezeichnet λ_{B} die Bragg-Wellenlänge, und Δλ_{B} die Änderung der Bragg-Wellenlänge. ε bezeichnet die (dimensionslose) Dehnung oder Stauchung des Glasfasermaterials, und ΔT bezeichnet die Temperaturänderung, α den thermischen Ausdehnungskoeffizienten, pₑ den effektiven elektrooptischen Koeffizienten (0,211) und ξ den thermooptischen Koeffizienten.

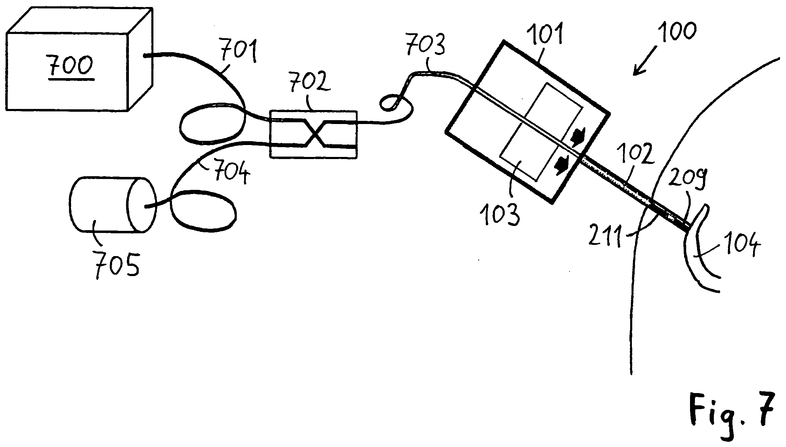

In Fig. 7 ist das Indentierungsmessgerät 100 zusammen mit einer zugehörigen optischen Messanordnung gezeigt. Das Indentierungsmessgerät 100 umfasst das Gehäuse 101 sowie das stabförmige Messelement 102 mit der Messsonde, die durch das im Gehäuse 101 angeordnete piezoelektrische Stellelement 103 vor- und zurückbewegbar ist. Die Messsonde umfasst eine Glasfaser mit den beiden Faser-Bragg-Gittern 209 und 211.

Der optische Messaufbau, der häufig auch als "optischer Interrogator" bezeichnet wird, umfasst eine Lichtquelle 700, die über eine optische Faser 701, einen Faserkoppler 702 und eine optische Faser 703 Licht in das Indentierungsmessgerät 100 einspeist. Als Lichtquelle 700 kann ein durchstimmbarer Laser eingesetzt werden, wobei die Frequenz des emittierten Laserlichts innerhalb eines bestimmten Frequenzbereichs periodisch durchgefahren wird. Alternativ dazu kann als Lichtquelle 700 eine Weißlichtquelle verwendet werden, deren Licht ein ganzes Frequenzband von unterschiedlichen spektralen Lichtkomponenten enthält. Das emittierte Licht wird über die optische Faser 701, den Faserkoppler 702 und die optische Faser 703 in das Indentierungsmessgerät 100 eingespeist. Das erste Faser-Bragg-Gitter 209 reflektiert das einfallende Licht bei einer ersten Bragg-Wellenlänge, während das als Referenz dienende zweite Faser-Bragg-Gitter 211 Licht bei einer zweiten Bragg-Wellenlänge reflektiert. Die rückreflektierten spektralen Komponenten gelangen über die optische Faser 703, den Faserkoppler 702 und eine optische Faser 704 zu einer Detektionseinheit 705, die beispielsweise mittels einer Fotodiode realisiert sein kann. Die Detektionseinheit 705 ist dazu ausgelegt, die Spektralkomponenten des rückreflektierten Signals auszuwerten. Anhand der Wellenlängen der rückreflektierten Spektralkomponenten kann die auf die Messsonde einwirkende Kraft als Funktion der Zeit verfolgt und aufgezeichnet werden.

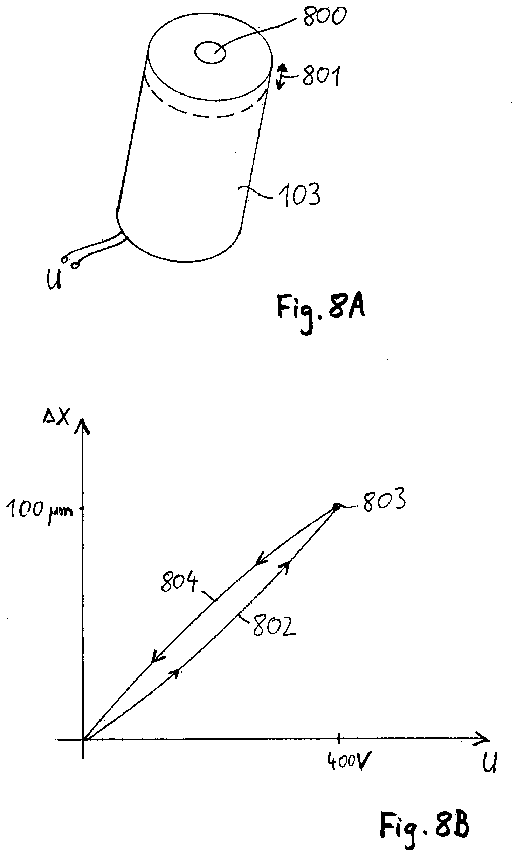

In Fig. 8A ist das piezoelektrische Stellelement 103 dargestellt. Das piezoelektrische Stellelement 103 ist als zylinderförmiges Piezoelement ausgebildet, welches ein Loch 800 für die Durchführung der zweiten Glasfaser 214 aufweist. Das piezoelektrische Stellelement 103 dient als Stellglied für das Röhrchen 202 und die darin befestigte erste Glasfaser 206. Wenn an das piezoelektrische Stellelement 103 z.B. eine Spannung U im Bereich bis zu 400 V angelegt wird, so führt dies zu einer entsprechenden Ausdehnung des piezoelektrischen Stellelements 103 in lateraler Richtung, wie dies durch den Pfeil 801 in Fig. 8A veranschaulicht ist. Über die angelegte Spannung U lässt sich der Vorschub des Röhrchens 202 und der darin angebrachten ersten Glasfaser 206 mit hoher Genauigkeit steuern.

Der Zusammenhang zwischen der Spannung U und der Ausdehnung Δx des piezoelektrischen Stellelements 103 ist in Fig. 8B gezeigt. Entlang der Rechtsachse ist die angelegte Spannung U aufgetragen, die sich z.B. im Bereich zwischen 0 V und 400 V bewegt. Entlang der Hochachse ist die durch die Spannung bewirkte Auslenkung Δx des piezoelektrischen Stellelements 103 aufgetragen, welche sich beispielsweise im Bereich zwischen 0 und 100 µm bewegen kann. Wenn die Spannung von 0 V auf einen Wert von z.B. einige zehn Volt oder einige hundert Volt erhöht wird, dann folgt die Ausdehnung des Piezos der Kurve 802. Wenn die Ausdehnung Δx am Punkt 803 einen gewissen Wert erreicht hat und die Spannung dann wieder reduziert wird, dann folgt die Ausdehnung des piezoelektrischen Stellelements 103 der Kurve 804, die sich von der Kurve 802 unterscheidet. Das Ausdehnungsverhalten des piezoelektrischen Stellelements 103 weist also eine gewisse Hysterese auf.

Um die durch diese Hysterese verursachte Beeinträchtigung der Stellgenauigkeit zumindest zum Teil auszugleichen, kann die am Piezo anliegende Spannung mittels eines Regelkreises geregelt werden. Dabei kann die aktuelle Position des Piezo beispielsweise mittels eines Dehnungsmessstreifens oder mittels eines kapazitiven Abstandssensors oder mittels eines dritten Faser-Bragg-Gitters erfasst werden, und in Abhängigkeit von dieser aktuellen Position wird die angelegte Spannung mittels des Regelkreises so nachgeregelt, dass eine gewünschte Sollposition erreicht wird. Durch den Einsatz eines derartigen rückgekoppelten piezoelektrischen Stellelements, welches mittels eines Regelkreises betrieben wird, lässt sich die ohnehin gute Stellgenauigkeit des piezoelektrischen Stellelements noch weiter verbessern.

Das piezoelektrische Stellelement 103 kann als einzelnes Element aus piezokeramischem Material ausgebildet sein. Alternativ dazu kann das piezoelektrische Stellelement 103 aus einem Stapel (Stack) von mehreren einzelnen Stapelelementen aus piezokeramischem Material aufgebaut sein, wobei jedes Stapelelement einzeln ansteuerbar ist und wobei sich die Ausdehnungen der einzelnen Stapelelemente addieren. Die Stapelelemente werden dabei in der Regel alle mit derselben Spannung beaufschlagt. Die Vorteile eines aus einem Stapel von mehreren Stapelelementen aufgebauten Piezos gegenüber einem einstückigen Piezo sind ein größerer Stellweg, eine verbesserte Positioniergenauigkeit sowie eine geringere Hysterese.

Damit eine beginnende Knorpeldegeneration in einer nur wenige µm dicken oberflächennahen Knorpelschicht erkannt werden kann, sollte sich auch die Indentierungstiefe im Bereich von einigen µm bewegen. Dies ist mit einem piezoelektrischen Stellelement 103 ohne Probleme möglich. Wenn an das piezoelektrische Stellelement 103 beispielsweise Spannungen im Bereich von 0 bis ca. 40 V angelegt werden, erhält man eine zugehörige Indentierungstiefe von 0 µm bis ca. 10 µm mit einer Stellgenauigkeit im Sub-Mikrometerbereich. Mit einer Ansteuerspannung U in diesem Spannungsbereich lassen sich daher die benötigten Indentierungstiefen von weniger als 10 µm realisieren, die zur Erkennung der Arthrose im Frühstadium benötigt werden.

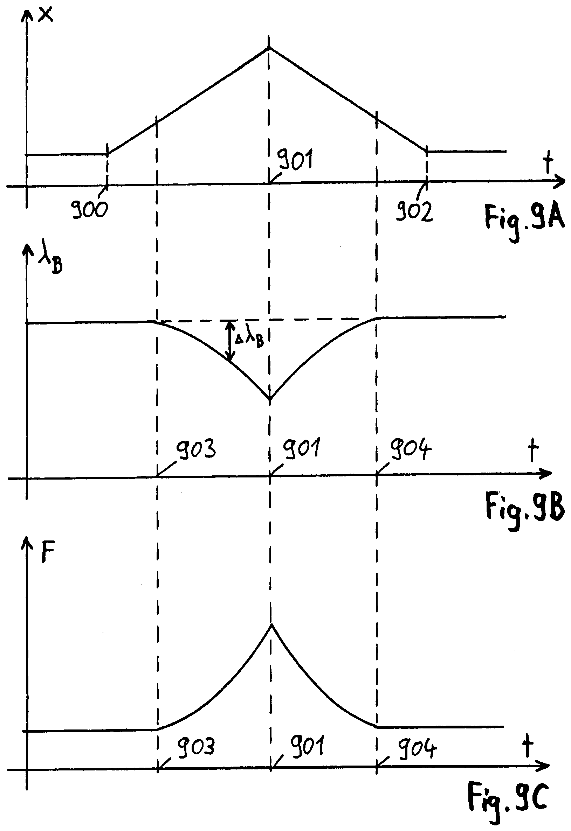

Der Ablauf einer Indentierungsmessung ist in den Fig. 9A bis 9C veranschaulicht. Fig. 9A zeigt die Auslenkung x des piezoelektrischen Stellelements als Funktion der Zeit. Zwischen den Zeitpunkten 900 und 901 wird die am piezoelektrischen Stellelement 103 anliegende Spannung erhöht, so dass man eine lineare Auslenkung des piezoelektrischen Stellelements erhält. Zwischen den Zeitpunkten 900 und 901 wird daher das Röhrchen 202 mit der darin befestigten ersten Glasfaser 206 gleichmäßig nach vorne geschoben. Zwischen den Zeitpunkten 901 und 902 wird die am piezoelektrischen Stellelement 103 anliegende Spannung wieder reduziert, die Auslenkung des Piezos wird wieder linear zurückgefahren.

Die während des Vortriebs des Piezos auf die Messspitze wirkende Kraft wird mittels des ersten Faser-Bragg-Gitters 209 erfasst. Wenn eine Kraft auf die Messspitze einwirkt, wird das erste Faser-Bragg-Gitter 209 dementsprechend gestaucht, und die Bragg-Wellenlänge λ_{B} verschiebt sich zu kleineren Wellenlängen hin. In Fig. 9B ist zu der in Fig. 9A gezeigten Piezoauslenkung ein möglicher zugehöriger Verlauf der Bragg-Wellenlänge λ_{B} des ersten Faser-Bragg-Gitters 209 als Funktion der Zeit aufgetragen. Die Bragg-Wellenlänge λ_{B} des ersten Faser-Bragg-Gitters 209 bleibt bis zum Zeitpunkt 903 konstant. Der Zeitpunkt 903 bezeichnet den sogenannten Kontaktpunkt, bei dem die Spitze der vorgeschobenen Messsonde die Oberfläche des Knorpelgewebes erreicht. Zwischen dem Zeitpunkt 903 und dem Zeitpunkt 901 wird die Messsonde noch weiter vorgeschoben und somit in das Knorpelgewebe eingedrückt. Dementsprechend beginnt eine immer größer werdende Indentierungskraft auf die Messsondenspitze zu wirken. Dies führt zu einer entsprechenden Stauchung des ersten Faser-Bragg-Gitters 209. Das erste Faser-Bragg-Gitter 209 wird infolge der einwirkenden Kraft gestaucht, und dementsprechend verringert sich die Bragg-Wellenlänge λ_{B} des rückreflektierten Lichts. Die zunehmende Verringerung der Bragg-Wellenlänge λ_{B} zwischen den Zeitpunkten 903 und 901 ist in Fig. 9B zu erkennen. Ab dem Zeitpunkt 901 wird die Messsonde wieder zurückbewegt. Dementsprechend verringert sich die auf das erste Faser-Bragg-Gitter 209 einwirkende Kraft, und zwischen den Zeitpunkten 901 und 904 kehrt die Bragg-Wellenlänge λ_{B} wieder zu ihrem ursprünglichen Wert zurück.

In Fig. 9C ist die auf die Spitze der Sonde einwirkende Kraft F als Funktion der Zeit dargestellt. Je geringer die in Fig. 9B dargestellte Bragg-Wellenlänge λ_{B} ist, desto höher ist die auf die Sondenspitze einwirkende Kraft F. Es muss kein linearer Zusammenhang zwischen dem Vorschub und der Kraft bestehen.

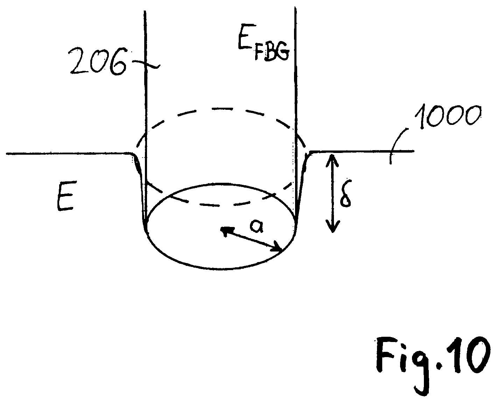

Aus dem in den Fig. 9A bis 9C gezeigten Zusammenhang zwischen Vorschub und Kraft können Messparameter, wie möglicherweise ein Elastizitätsmodul E des untersuchten Gewebes, abgeleitet werden. Im Folgenden soll das zur Berechnung verwendete Modell kurz diskutiert werden. Die hierzu verwendete Terminologie ist anhand von Fig. 10 veranschaulicht.

Fig. 10 zeigt, wie die Spitze der ersten Glasfaser 206 in das zu untersuchende Gewebe 1000 eingedrückt wird. Der Radius an der Spitze der ersten Glasfaser 206 ist mit a bezeichnet, und die Tiefe der Indentierung wird mit δ bezeichnet. Infolge der Eindrückung wirkt eine Kraft F auf die Spitze der ersten Glasfaser 206 ein. Infolge dieser Indentierungskraft F kommt es zu einer entsprechenden Verschiebung Δλ_{B} der Bragg-Wellenlänge. Das (vorbekannte) Elastizitätsmodul der ersten Glasfaser 206 wird mit E_{FBG} bezeichnet, wohingegen das zu bestimmende Elastizitätsmodul des untersuchten Gewebes 1000 mit E bezeichnet ist.

Zunächst soll der Zusammenhang zwischen der Indentierungskraft F, der Stauchung der ersten Glasfaser 206 und der dadurch bewirkten Verstimmung Δλ_{B} der Bragg-Wellenlänge untersucht werden. Für den Zusammenhang zwischen der Indentierungskraft F und der Dehnung bzw. Stauchung ε kann möglicherweise der Zusammenhang gelten: $F = ε ⋅ π ⋅ {a}^{2} ⋅ {E}_{FBG}$ (3) wobei ε die Stauchung der ersten Glasfaser 206 bezeichnet, a den Radius an der Spitze der ersten Glasfaser 206 und E_{FBG} das Elastizitätsmodul der ersten Glasfaser 206 bezeichnet.

Darüber hinaus besteht ein Zusammenhang zwischen der Dehnung bzw. Stauchung ε der ersten Glasfaser 206 und der dadurch bewirkten Verschiebung Δλ_{B} der Bragg-Wellenlänge: $\frac{{Δλ}_{B}}{{λ}_{B}} = \left(1-{p}_{e}\right) ⋅ ε$ (4) wobei pₑ den effektiven elastooptischen Koeffizienten bezeichnet (ca. 0,211 für eine Faser vom Typ GF1B). Wenn man diese Beziehung nach ε auflöst und in Gleichung (3) einsetzt, dann erhält man für die Kraft F: $F = \frac{π ⋅ {a}^{2}}{\left(1-{p}_{e}\right)} ⋅ {E}_{FBG} ⋅ \frac{{Δλ}_{B}}{{λ}_{B}}$ (5)

Es ergibt sich also eine direkte Proportionalität zwischen Δλ_{B} und der Indentierungskraft F.

Die Indentierungskraft F lässt sich unter bestimmten Bedingungen mittels des Hertz-Modells auch aus der Sicht des eingedrückten Gewebes beschreiben. Dabei ergibt sich für die Indentierungskraft F $F = \frac{2 ⋅ a ⋅ E ⋅ δ ⋅ κ}{\left(1-{ν}^{2}\right)}$ (6) wobei E ein Elastizitätsmodul des untersuchten Gewebes, a den Radius an der Spitze der Glasfaser und δ die Indentierungstiefe bezeichnet. Der Koeffizient κ bezeichnet einen durch eine Finite-Elemente-Methode (FEM) ermittelten Koeffizienten, der zu κ=1,218 ermittelt wurde. Im Nenner von Gleichung (6) taucht die Querkontraktionszahl bzw. Poissonzahl v auf, wobei hier v=0,185 gesetzt werden kann.

Man kann die Indentierungskraft jetzt also zum einen mit Hilfe von Gleichung (5) über das Elastizitätsmodul E_{FBG} der Glasfaser und die Verstimmung Δλ_{B} der Bragg-Wellenlänge ausdrücken. Zum andern kann man die Indentierungskraft F entsprechend Gleichung (6) mittels des Hertz-Modells über das Elastizitätsmodul E des untersuchten Gewebes und die Indentierungstiefe δ ausdrücken. Wenn man die beiden Ausdrücke für die Indentierungskraft F gleichsetzt, ergibt sich $F = \frac{2 ⋅ a ⋅ E ⋅ δ ⋅ κ}{\left(1-{ν}^{2}\right)} = \frac{π ⋅ {a}^{2}}{\left(1-{p}_{e}\right)} ⋅ {E}_{FBG} ⋅ \frac{{Δλ}_{B}}{{λ}_{B}}$ (7)

Aufgelöst nach dem Elastizitätsmodul E des untersuchten Gewebes ergibt sich $E = \frac{\left(1-{ν}^{2}\right)}{2 ⋅ a ⋅ δ ⋅ κ} ⋅ \frac{π ⋅ {a}^{2}}{\left(1-{p}_{e}\right)} ⋅ {E}_{FBG} ⋅ \frac{{Δλ}_{B}}{{λ}_{B}}$ (8)

Mit Hilfe dieser Formel lässt sich aus den in Fig. 9A bis 9C gezeigten Indentierungskurven ein Elastizitätsmodul E für das untersuchte Gewebe bestimmen. Die Bestimmung eines Elastizitätsmoduls kann für bestimmte Parameterbereiche und Indentierungstiefen ein sinnvolles Hilfsmittel für die Beschreibung der elastischen oder viskoelastischen Eigenschaften des Substrats sein.

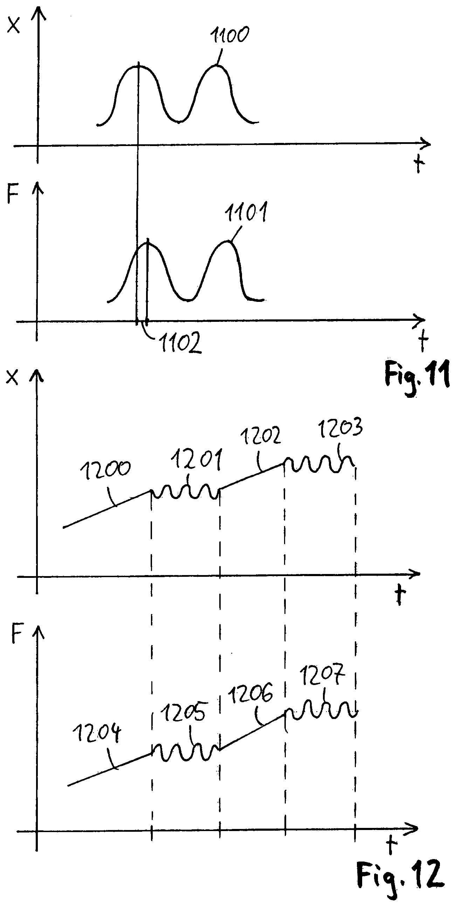

In Fig. 11 ist ein weiteres Messverfahren zur Ermittlung der elastischen oder viskoelastischen Eigenschaften eines Gewebes gezeigt, bei dem das piezoelektrische Stellelement 103 mit einer Sinusspannung beaufschlagt wird. Wie im oberen Diagramm von Fig. 11 gezeigt, ergibt sich dadurch eine sinusförmige Variation 1100 der Position x der Messspitze. Wenn man die auf die Messspitze einwirkende Indentierungskraft als Funktion der Zeit aufzeichnet, ergibt sich die im unteren Diagramm von Fig. 11 gezeigte sinusförmige Variation 1101 der Indentierungskraft, die eine gewisse Phasenverschiebung 1102 gegenüber der Position x aufweist. Diese Phasenverschiebung 1102 beschreibt das elastische Response-Verhalten des jeweiligen Gewebes und kann als Funktion der Frequenz der Sinusanregung erfasst werden. Dazu kann die Frequenz der Sinusanregung im Bereich von beispielsweise 0,5 bis 100 Hz durchgestimmt werden. Da insbesondere bei einem Knorpelgewebe mit Relaxationszeiten im Bereich von Sekunden zu rechnen ist, dürften signifikante Aussagen über das Relaxationsverhalten des Knorpelgewebes bei Anregungsfrequenzen insbesondere im Bereich von 0,5 bis 2 Hz erhalten werden.

In Fig. 12 ist ein weiteres Anregungsmuster für die Erfassung von elastischen Eigenschaften eines Gewebes gezeigt. Im oberen Teil von Fig. 12 ist die Position x der Sondenspitze als Funktion der Zeit t aufgetragen. Im Bereich 1200 ergibt sich ein linearer Vorschub der Sondenspitze, anschließend wird die Position der Sondenspitze im Bereich 1201 sinusförmig variiert. Im Bereich 1202 schließt sich ein weiterer linearer Vorschub der Sondenspitze an, und daraufhin wird im Bereich 1203 erneut eine sinusförmige Variation auf die Position x aufmoduliert.

Im unteren Teil von Fig. 12 ist der zugehörige Verlauf der Indentierungskraft F als Funktion der Zeit aufgetragen. In den Bereichen 1204 und 1206 ergibt sich jeweils ein linearer Anstieg der Indentierungskraft mit der Zeit. In den Bereichen 1205 und 1207 ergibt sich jeweils eine sinusförmige Variation der Indentierungskraft F, die jeweils eine gewisse Phasenverschiebung zur zugrundeliegenden sinusförmigen Variation der Position x aufweist. Mit Hilfe des in Fig. 12 gezeigten Anregungsschemas kann ein Elastizitätsmodul E des untersuchten Gewebes ermittelt werden. Darüber hinaus ermöglicht die sinusförmige Variation der Position x und die Erfassung der zugehörigen Indentierungskraft F eine Aussage über das zeitliche Relaxationsverhalten des Knorpelgewebes.

## Patentansprüche

1. Indentierungsvorrichtung (100) zur Bestimmung von elastischen oder viskoelastischen Eigenschaften von Knorpelgewebe, wobei die Indentierungsvorrichtung (100) aufweist
- eine Messsonde zur Indentierung eines Gewebes (1000), welche eine optische Faser (206) umfasst,
- mindestens ein in die optische Faser (206) eingeschriebenes Faser-Bragg-Gitter (209, 211), das dazu vorgesehen ist, eine auf die Spitze der optischen Faser (206) einwirkende Kraft (210) zu detektieren,
- wobei die Indentierungsvorrichtung (100) dazu ausgelegt ist, den Vorschub der Messsonde und die auf die Spitze der optischen Faser (206) einwirkende Kraft (210) zu erfassen, wobei
- die Messsonde ein Röhrchen (202) umfasst, innerhalb dessen die optische Faser (206) befestigt ist, wobei das Röhrchen (202) einen Außendurchmesser von weniger als 1 Millimeter aufweist,
- wobei die Indentierungsvorrichtung (100) ein piezoelektrisches Stellelement (103) zur Positionierung der Messsonde aufweist, das mit der Messsonde mechanisch verbunden ist und das dazu ausgelegt ist, die Messsonde auf das zu vermessende Gewebe (1000) zu zu bewegen und in das Gewebe (1000) einzudrücken,
- wobei die Indentierungsvorrichtung (100) dazu ausgelegt ist, die Spitze der optischen Faser (206) in das zu untersuchende Gewebe (1000) einzudrücken.

2. Indentierungsvorrichtung nach Anspruch 1, **gekennzeichnet durch** mindestens eines von folgenden:
- die optische Faser ist in das Röhrchen der Messsonde eingeschoben und am Röhrchen befestigt;
- das Röhrchen der Messsonde ist dazu ausgelegt, die im Inneren des Röhrchens befestigte optische Faser zu stabilisieren und zu fixieren;
- bei dem Röhrchen der Messsonde handelt es sich um ein Metallröhrchen oder ein Glasröhrchen;
- das Röhrchen der Messsonde weist einen Außendurchmesser im Bereich von ca. 200 Mikrometer bis 500 Mikrometer auf;
- das Röhrchen der Messsonde weist einen Außendurchmesser im Bereich von ca. 200 Mikrometer bis 300 Mikrometer auf;
- das Röhrchen der Messsonde weist einen Innendurchmesser im Bereich von ca. 90 Mikrometer bis 170 Mikrometer auf;
- die optische Faser weist einen Außendurchmesser im Bereich von ca. 80 Mikrometer bis 125 Mikrometer auf.

3. Indentierungsvorrichtung nach Anspruch 1 oder Anspruch 2, **dadurch gekennzeichnet, dass** die Indentierungsvorrichtung zusätzlich ein äußeres Führungsröhrchen für die Messsonde aufweist, wobei die Messsonde im äußeren Führungsröhrchen verschieblich aufgenommen ist.

4. Indentierungsvorrichtung nach einem der Ansprüche 1 bis 3, **gekennzeichnet durch** mindestens eines von folgenden:
- das piezoelektrische Stellelement ist dazu ausgelegt, die Messsonde in Richtung auf das Gewebe zu und vom Gewebe weg zu positionieren;
- das piezoelektrische Stellelement ist dazu ausgelegt, die Messsonde auf das zu vermessende Gewebe zu und vom Gewebe weg zu bewegen;
- das piezoelektrische Stellelement ist dazu ausgelegt, die Messsonde in Richtung auf das Gewebe zu und vom Gewebe weg zu bewegen und dabei in das Gewebe einzudrücken;
- das piezoelektrische Stellelement ist dazu ausgelegt, in Abhängigkeit von einem Hochspannungssignal die Messsonde in Richtung auf das Gewebe zu und vom Gewebe weg zu positionieren;
- zur Steuerung des Vorschubs ist an das piezoelektrische Stellelement eine Spannung von bis zu einigen hundert Volt anlegbar, wobei durch die angelegte Spannung ein Vorschub der Messsonde von bis zu einigen hundert Mikrometer bewirkt wird;
- bei dem piezoelektrischen Stellelement handelt es sich um ein rückgekoppeltes piezoelektrisches Stellelement, das dazu ausgelegt ist, die am piezoelektrischen Stellelement anliegende Spannung in Abhängigkeit vom tatsächlichen Vorschub des piezoelektrischen Stellelements einzuregeln;
- die durch das piezoelektrische Stellelement bewirkte Positionierung der Messsonde erfolgt mit einer Genauigkeit im Submikrometerbereich;
- der Vorschub der Messsonde während einer Indentierungsmessung ist so einstellbar, dass eine Indentierungstiefe von weniger als 40 Mikrometer erzielt wird;
- der Vorschub der Messsonde während einer Indentierungsmessung ist so einstellbar, dass eine Indentierungstiefe von weniger als 20 Mikrometer erzielt wird;
- der Vorschub der Messsonde während einer Indentierungsmessung ist so einstellbar, dass eine Indentierungstiefe von weniger als 10 Mikrometer erzielt wird.

5. Indentierungsvorrichtung nach einem der Ansprüche 1 bis 4, **dadurch gekennzeichnet, dass** die optische Faser ein erstes Faser-Bragg-Gitter aufweist, das dazu ausgelegt ist, die auf die Spitze der optischen Faser einwirkende Kraft zu detektieren.

6. Indentierungsvorrichtung nach Anspruch 5, **gekennzeichnet durch** mindestens eines von folgenden:
- das erste Faser-Bragg-Gitter ist im Bereich der Spitze der optischen Faser angeordnet;
- die auf die Spitze der optischen Faser einwirkende Kraft verursacht eine Stauchung des ersten Faser-Bragg-Gitters;
- während der Indentierung des Gewebes durch die Messsonde wirkt auf die Spitze der optischen Faser eine Kraft ein, die eine entsprechende Stauchung des ersten Faser-Bragg-Gitters hervorruft;
- die auf die Spitze der optischen Faser einwirkende Kraft verursacht eine Stauchung des ersten Faser-Bragg-Gitters, wobei die Stauchung des ersten Faser-Bragg-Gitters eine entsprechende Verschiebung der Mittenwellenlänge des vom ersten Faser-Bragg-Gitter reflektierten Lichts zu kleineren Wellenlängen hin bewirkt;
- die auf die Spitze der optischen Faser einwirkende Kraft verursacht eine Stauchung des ersten Faser-Bragg-Gitters, wobei die Stauchung des ersten Faser-Bragg-Gitters eine entsprechende Verschiebung der Bragg-Wellenlänge des ersten Faser-Bragg-Gitters zu kleineren Wellenlängen hin bewirkt;
- anhand einer Verschiebung der Wellenlänge des vom ersten Faser-Bragg-Gitter rückreflektierten Lichts ist die auf die Spitze der optischen Faser einwirkende Kraft bestimmbar.

7. Indentierungsvorrichtung nach einem der Ansprüche 1 bis 6, **dadurch gekennzeichnet, dass** die optische Faser in das Röhrchen der Messsonde eingeschoben ist und an mindestens einer Verklebungsstelle mit dem Röhrchen verklebt ist.

8. Indentierungsvorrichtung nach Anspruch 7, **dadurch gekennzeichnet, dass** die optische Faser ein erstes Faser-Bragg-Gitter aufweist, das dazu ausgelegt ist, die auf die Spitze der optischen Faser einwirkende Kraft zu detektieren, wobei das erste Faser-Bragg-Gitter zwischen der vordersten der mindestens einen Verklebungsstelle und der Spitze der optischen Faser angeordnet ist.

9. Indentierungsvorrichtung nach einem der Ansprüche 1 bis 8, **gekennzeichnet durch** mindestens eines von folgenden:
- das piezoelektrische Stellelement ist dazu ausgelegt, die Messsonde während einer Indentierungsmessung mit einem linearen Vorschub in Richtung des Gewebes zu bewegen;
- die Indentierungsvorrichtung ist dazu ausgelegt, die während eines linearen Vorschubs der Messsonde auf die Spitze der optischen Faser einwirkende Kraft zu erfassen;
- die Indentierungsvorrichtung umfasst eine Spannungsversorgung, die dazu ausgelegt ist, die am piezoelektrischen Stellelement anliegende Spannung so zu erhöhen, dass die Messsonde mit einem linearen Vorschub auf das Gewebe zu bewegt und in das zu untersuchende Gewebe eingedrückt wird;
- die Indentierungsvorrichtung umfasst eine Spannungsversorgung, die dazu ausgelegt ist, dem piezoelektrischen Stellelement eine sägezahnförmige oder dreieckförmige Spannung zuzuführen.

10. Indentierungsvorrichtung nach einem der Ansprüche 1 bis 9, **dadurch gekennzeichnet, dass** die Indentierungsvorrichtung eine Auswerteeinheit umfasst, die dazu ausgelegt ist, die auf die optische Faser einwirkende Kraft in Abhängigkeit vom Vorschub der Messsonde zu erfassen.

11. Indentierungsvorrichtung nach einem der Ansprüche 1 bis 10, **gekennzeichnet durch** mindestens eines von folgenden:
- die Auswerteeinheit ist dazu ausgelegt, aus dem Vorschub der Messsonde und der auf die Spitze der optischen Faser einwirkenden Kraft elastische oder viskoelastische Eigenschaften des Gewebes zu ermitteln;
- die Auswerteeinheit ist dazu ausgelegt, aus dem Zusammenhang zwischen dem Vorschub der Messsonde und der auf die Spitze der optischen Faser einwirkenden Kraft ein Elastizitätsmodul des untersuchten Gewebes zu bestimmen.

12. Indentierungsvorrichtung nach einem der Ansprüche 1 bis 11, **dadurch gekennzeichnet, dass** die Indentierungsvorrichtung eine Spannungsversorgung umfasst, die dazu ausgelegt ist, der am piezoelektrischen Stellelement anliegenden Spannung eine oszillierende Spannung zu überlagern, die eine oszillierende Bewegung der in das Gewebe eingedrückten Messsonde verursacht.

13. Indentierungsvorrichtung nach einem der Ansprüche 1 bis 12, **gekennzeichnet durch** mindestens eines von folgenden:
- bei dem Gewebe handelt es sich um Knorpelgewebe;
- bei dem Gewebe handelt es sich um Knorpelgewebe, wobei die Indentierungsvorrichtung dazu ausgelegt ist, die elastischen oder viskoelastischen Eigenschaften des Knorpelgewebes zu bestimmen;
- die Indentierungsvorrichtung ist für eine minimalinvasive Diagnostik ausgelegt;
- die Indentierungsvorrichtung ist für eine Untersuchung von Gewebe in vivo ausgelegt;
- die Indentierungsvorrichtung ist für eine Untersuchung von Knorpelgewebe in vivo ausgelegt;
- bei dem Gewebe handelt es sich um Knorpelgewebe, wobei die Indentierungsvorrichtung dazu ausgelegt ist, anhand einer Veränderung von elastischen oder viskoelastischen Eigenschaften des Knorpelgewebes eine beginnende Degeneration des Knorpelgewebes festzustellen;
- die Indentierungsvorrichtung ist für eine Diagnose der Arthrose ausgelegt.

## Claims

1. An indentation device (100) for determining the elastic or viscoelastic properties of cartilage tissue, the indentation device (100) comprising
- a measuring probe for indenting a tissue (1000), comprising an optical fiber (206),
- at least one fiber Bragg grating (209, 211) inscribed in the optical fiber (206) and designed to detect a force (210) acting on the tip of the optical fiber (206),
- wherein the indentation device (100) is configured to capture the displacement of the measuring probe and the force (210) acting on the tip of the optical fiber (206),
- wherein the measuring probe comprises a tube (202) within which the optical fiber (206) is secured, and the tube (202) has an outer diameter of less than 1 millimeter,
- wherein the indentation device (100) comprises a piezoelectric control element (103) for positioning the measuring probe, said control element being mechanically connected to the measuring probe and configured to displace the measuring probe toward the tissue (1000) to be measured and to press said probe into the tissue (1000),
- wherein the indentation device (100) is configured to press the tip of the optical fiber (206) into the tissue (1000) to be examined.

2. The indentation device according to claim 1, **characterized by** at least one of the following:
- the optical fiber is inserted into the tube of the measuring probe and attached to the tube;
- the measuring probe tube is configured to stabilize and secure the optical fiber attached inside the tube;
- the measuring probe tube is either a metal tube or a glass tube;
- the measuring probe tube has an outer diameter ranging from approximately 200 micrometers to 500 micrometers;
- the measuring probe tube has an outer diameter ranging from approximately 200 micrometers to 300 micrometers;
- the measuring probe tube has an inner diameter ranging from approximately 90 micrometers to 170 micrometers;
- the optical fiber has an outer diameter ranging from approximately 80 micrometers to 125 micrometers.

3. The indentation device according to claim 1 or claim 2, **characterized in that** the indentation device further comprises an outer guide tube for the measuring probe, wherein the measuring probe is displaceably received within the outer guide tube.

4. The indentation device according to any one of the claims 1 through 3, **characterized by** at least one of the following:
- the piezoelectric control element is configured to position the measuring probe in the direction toward and away from the tissue;
- the piezoelectric control element is configured to displace the measuring probe toward and away from the tissue to be measured;
- the piezoelectric control element is configured to displace the measuring probe in the direction toward and away from the tissue, pressing said probe into the tissue in the process;
- the piezoelectric control element is configured to position the probe in the direction toward and away from the tissue in response to a high-voltage signal;
- to control the displacement, a voltage of up to several hundred volts can be applied to the piezoelectric control element, wherein the applied voltage causes the measuring probe to be displaced forward by up to several hundred micrometers;
- the piezoelectric control element is a feedback-controlled piezoelectric control element configured to regulate the voltage applied to the piezoelectric control element as a function of the actual displacement of the piezoelectric control element;
- the positioning of the measuring probe brought about by the piezoelectric control element is carried out with submicrometer accuracy;
- the displacement of the measuring probe during an indentation measurement can be adjusted to achieve an indentation depth of less than 40 micrometers;
- the displacement of the measuring probe during an indentation measurement can be adjusted to achieve an indentation depth of less than 20 micrometers;
- the displacement of the measuring probe during an indentation measurement can be adjusted to achieve an indentation depth of less than 10 micrometers.

5. The indentation device according to any one of the claims 1 through 4, **characterized in that** the optical fiber comprises a first fiber Bragg grating configured to detect the force acting on the tip of the optical fiber.

6. The indentation device according to claim 5, **characterized by** at least one of the following:
- the first fiber Bragg grating is disposed near the tip of the optical fiber;
- the force acting on the tip of the optical fiber causes the first fiber Bragg grating to compress;
- as the measuring probe indents the tissue, a force acts on the tip of the optical fiber, causing a corresponding compression of the first fiber Bragg grating;
- the force acting on the tip of the optical fiber causes compression of the first fiber Bragg grating, wherein the compression of the first fiber Bragg grating causes a corresponding shift in the center wavelength of the light reflected by the first fiber Bragg grating toward shorter wavelengths;
- the force acting on the tip of the optical fiber causes compression of the first fiber Bragg grating, wherein the compression of the first fiber Bragg grating causes a corresponding shift of the Bragg wavelength of the first fiber Bragg grating toward shorter wavelengths;
- the force acting on the tip of the optical fiber can be determined based on a shift in the wavelength of the light reflected by the first fiber Bragg grating.

7. The indentation device according to any one of the claims 1 through 6, **characterized in that** the optical fiber is inserted into the tube of the measuring probe and is bonded to the tube at at least one bonding point.

8. The indentation device according to claim 7, **characterized in that** the optical fiber comprises a first fiber Bragg grating configured to detect the force acting on the tip of the optical fiber, wherein the first fiber Bragg grating is disposed between the foremost of the at least one bonding point and the tip of the optical fiber.

9. The indentation device according to any one of the claims 1 through 8, **characterized by** at least one of the following:
- the piezoelectric control element is configured to displace the measuring probe toward the tissue at a linear displacement during an indentation measurement;
- the indentation device is configured to capture the force acting on the tip of the optical fiber during linear displacement of the measuring probe;
- the indentation device comprises a power supply configured to increase the voltage applied to the piezoelectric control element so that the measuring probe moves toward the tissue at a linear displacement and is pressed into the tissue to be examined;
- the indentation device comprises a power supply configured to supply a sawtooth-shaped or triangular voltage to the piezoelectric control element.

10. The indentation device according to any one of the claims 1 through 9, **characterized in that** the indentation device comprises an evaluation unit configured to capture the force acting on the optical fiber as a function of the displacement of the measuring probe.

11. The indentation device according to any one of the claims 1 through 10, **characterized by** at least one of the following:
- the evaluation unit is configured to determine the elastic or viscoelastic properties of the tissue based on the displacement of the measuring probe and the force acting on the tip of the optical fiber;
- the evaluation unit is configured to determine the modulus of elasticity of the examined tissue based on the relationship between the displacement of the measuring probe and the force acting on the tip of the optical fiber.

12. The indentation device according to any one of the claims 1 through 11, **characterized in that** the indentation device comprises a power supply configured to superimpose an oscillating voltage on the voltage applied to the piezoelectric control element, thereby causing the measuring probe pressed into the tissue to oscillate.

13. The indentation device according to any one of the claims 1 through 12, **characterized by** at least one of the following:
- the tissue in question is cartilage;
- the tissue in question is cartilage, and the indentation device is configured to determine the elastic or viscoelastic properties of the cartilage;
- the indentation device is configured for minimally invasive diagnostics;
- the indentation device is configured for the in vivo examination of tissue;
- the indentation device is configured for the in vivo examination of cartilage tissue;
- the tissue in question is cartilage, and the indentation device is configured to determine the onset of cartilage degeneration based on a change in the elastic or viscoelastic properties of the cartilage;
- the indentation device is configured to diagnose osteoarthritis.

## Revendications

1. Dispositif d'indentation (100) destiné à déterminer des propriétés élastiques ou viscoélastiques du tissu cartilagineux, le dispositif d'indentation (100) comportant
- une sonde de mesure destinée à l'indentation d'un tissu (1000), laquelle comprend une fibre optique (206),
- au moins un réseau de Bragg sur fibre (209, 211) gravé dans la fibre optique (206), qui est destiné à détecter une force (210) agissant sur la pointe de la fibre optique (206),
- le dispositif d'indentation (100) étant conçu pour détecter l'avance de la sonde de mesure et la force (210) agissant sur la pointe de la fibre optique (206),
- la sonde de mesure comprenant un petit tube (202) à l'intérieur duquel est fixée la fibre optique (206), le petit tube (202) présentant un diamètre extérieur inférieur à 1 millimètre,
- le dispositif d'indentation (100) comportant un élément de commande piézoélectrique (103) destiné à positionner la sonde de mesure, qui est relié mécaniquement à la sonde de mesure et est conçu pour déplacer la sonde de mesure vers le tissu (1000) à mesurer et pour l'enfoncer dans le tissu (1000),
- le dispositif d'indentation (100) étant conçu pour enfoncer la pointe de la fibre optique (206) dans le tissu (1000) à examiner.

2. Dispositif d'indentation selon la revendication 1, **caractérisé par** au moins l'un des éléments suivants :
- la fibre optique est insérée dans le petit tube de la sonde de mesure et fixée au petit tube ;
- le petit tube de la sonde de mesure est conçu pour stabiliser et maintenir en place la fibre optique fixée à l'intérieur du petit tube ;
- le petit tube de la sonde de mesure est un petit tube métallique ou un petit tube en verre ;
- le petit tube de la sonde de mesure présente un diamètre extérieur compris dans la plage d'environ 200 micromètres à 500 micromètres ;
- le petit tube de la sonde de mesure présente un diamètre extérieur compris dans la plage d'environ 200 micromètres à 300 micromètres ;
- le petit tube de la sonde de mesure présente un diamètre intérieur compris dans la plage d'environ 90 micromètres à 170 micromètres ;
- la fibre optique présente un diamètre extérieur compris dans la plage d'environ 80 micromètres à 125 micromètres.

3. Dispositif d'indentation selon la revendication 1 ou la revendication 2, **caractérisé en ce que** le dispositif d'indentation comporte en outre un petit tube de guidage extérieur pour la sonde de mesure, la sonde de mesure étant logée de manière coulissante dans le petit tube de guidage extérieur.

4. Dispositif d'indentation selon l'une des revendications 1 à 3, **caractérisé par** au moins l'un des éléments suivants :
- l'élément de commande piézoélectrique est conçu pour positionner la sonde de mesure en direction du tissu et à l'écart du tissu ;
- l'élément de commande piézoélectrique est conçu pour déplacer la sonde de mesure vers le tissu à mesurer et l'éloigner du tissu ;
- l'élément de commande piézoélectrique est conçu pour déplacer la sonde de mesure en direction du tissu et l'éloigner du tissu, tout en l'enfonçant dans le tissu ;
- l'élément de commande piézoélectrique est conçu pour positionner la sonde de mesure en direction du tissu et à l'écart du tissu en fonction d'un signal haute tension ;
- pour commander l'avance, une tension pouvant atteindre quelques centaines de volts peut être appliquée à l'élément de commande piézoélectrique, la tension appliquée provoquant une avance de la sonde de mesure pouvant atteindre quelques centaines de micromètres ;
- l'élément de commande piézoélectrique est un élément de commande piézoélectrique à rétroaction, qui est conçu pour réguler la tension appliquée à l'élément de commande piézoélectrique en fonction de l'avance réelle de l'élément de commande piézoélectrique ;
- le positionnement de la sonde de mesure, produit par l'élément de commande piézoélectrique, s'effectue avec une précision dans la plage submicronique ;
- l'avance de la sonde de mesure pendant une mesure d'indentation peut être réglée de manière à permettre d'obtenir une profondeur d'indentation inférieure à 40 micromètres ;
- l'avance de la sonde de mesure pendant une mesure d'indentation peut être réglée de manière à permettre d'obtenir une profondeur d'indentation inférieure à 20 micromètres ;
- l'avance de la sonde de mesure pendant une mesure d'indentation peut être réglée de manière à permettre d'obtenir une profondeur d'indentation inférieure à 10 micromètres.

5. Dispositif d'indentation selon l'une des revendications 1 à 4, **caractérisé en ce que** la fibre optique comporte un premier réseau de Bragg sur fibre, qui est conçu pour détecter la force agissant sur la pointe de la fibre optique.

6. Dispositif d'indentation selon la revendication 5, **caractérisé par** au moins l'un des éléments suivants :
- le premier réseau de Bragg sur fibre est disposé dans la zone de la pointe de la fibre optique ;
- la force agissant sur la pointe de la fibre optique provoque un tassement du premier réseau de Bragg sur fibre ;
- pendant l'indentation du tissu par la sonde de mesure, une force agit sur la pointe de la fibre optique, laquelle provoque un tassement correspondant du premier réseau de Bragg sur fibre ;
- la force agissant sur la pointe de la fibre optique provoque un tassement du premier réseau de Bragg sur fibre, le tassement du premier réseau de Bragg sur fibre entraînant un décalage correspondant de la longueur d'onde centrale de la lumière réfléchie par le premier réseau de Bragg sur fibre vers des longueurs d'onde plus courtes ;
- la force agissant sur la pointe de la fibre optique provoque un tassement du premier réseau de Bragg sur fibre, le tassement du premier réseau de Bragg sur fibre entraînant un décalage correspondant de la longueur d'onde de Bragg du premier réseau de Bragg sur fibre vers des longueurs d'onde plus courtes ;
- la force agissant sur la pointe de la fibre optique peut être déterminée à l'aide d'un décalage de la longueur d'onde de la lumière rétroréfléchie par le premier réseau de Bragg sur fibre.

7. Dispositif d'indentation selon l'une des revendications 1 à 6, **caractérisé en ce que** la fibre optique est insérée dans le petit tube de la sonde de mesure et est collée au petit tube en au moins un point de collage.

8. Dispositif d'indentation selon la revendication 7, **caractérisé en ce que** la fibre optique comporte un premier réseau de Bragg sur fibre, qui est conçu pour détecter la force agissant sur la pointe de la fibre optique, le premier réseau de Bragg sur fibre étant disposé entre le plus en avant de l'au moins un point de collage et la pointe de la fibre optique.

9. Dispositif d'indentation selon l'une des revendications 1 à 8, **caractérisé par** au moins l'un des éléments suivants :
- l'élément de commande piézoélectrique est conçu pour, pendant une mesure d'indentation, déplacer la sonde de mesure dans la direction du tissu avec une avance linéaire ;
- le dispositif d'indentation est conçu pour détecter la force agissant sur la pointe de la fibre optique pendant une avance linéaire de la sonde de mesure ;
- le dispositif d'indentation comprend une alimentation électrique qui est conçue pour accroître la tension appliquée à l'élément de commande piézoélectrique de manière à ce que la sonde de mesure soit déplacée vers le tissu avec une avance linéaire et enfoncée dans le tissu à examiner ;
- le dispositif d'indentation comprend une alimentation électrique qui est conçue pour fournir à l'élément de commande piézoélectrique une tension en dents de scie ou triangulaire.

10. Dispositif d'indentation selon l'une des revendications 1 à 9, **caractérisé en ce que** le dispositif d'indentation comprend une unité d'évaluation qui est conçue pour détecter la force agissant sur la fibre optique en fonction de l'avance de la sonde de mesure.

11. Dispositif d'indentation selon l'une des revendications 1 à 10, **caractérisé par** au moins l'un des éléments suivants :
- l'unité d'évaluation est conçue pour déterminer des propriétés élastiques ou viscoélastiques du tissu à l'aide de l'avance de la sonde de mesure et de la force agissant sur la pointe de la fibre optique ;
- l'unité d'évaluation est conçue pour déterminer un module d'élasticité du tissu examiné à l'aide de la relation entre l'avance de la sonde de mesure et la force agissant sur la pointe de la fibre optique.

12. Dispositif d'indentation selon l'une des revendications 1 à 11, **caractérisé en ce que** le dispositif d'indentation comprend une alimentation électrique qui est conçue pour superposer à la tension appliquée à l'élément de commande piézoélectrique une tension oscillante qui provoque un mouvement oscillant de la sonde de mesure enfoncée dans le tissu.

13. Dispositif d'indentation selon l'une des revendications 1 à 12, **caractérisé par** au moins l'un des éléments suivants :
- le tissu est un tissu cartilagineux ;
- le tissu est un tissu cartilagineux, le dispositif d'indentation étant conçu pour déterminer les propriétés élastiques ou viscoélastiques du tissu cartilagineux ;
- le dispositif d'indentation est conçu pour un diagnostic minimalement invasif ;
- le dispositif d'indentation est conçu pour un examen de tissus in vivo ;
- le dispositif d'indentation est conçu pour un examen in vivo du tissu cartilagineux ;
- le tissu est un tissu cartilagineux, le dispositif d'indentation étant conçu pour constater un début de dégénérescence du tissu cartilagineux à l'aide d'une modification de propriétés élastiques ou viscoélastiques du tissu cartilagineux ;
- le dispositif d'indentation est conçu pour un diagnostic de l'arthrose.
